# EUROPEAN PATENT APPLICATION

(11) **EP 2 182 058 A1**
(43) Date of publication of application: **05.05.2010**
(21) Application number: 08166956.6
(22) Date of filing: 17.10.2008
(51) Int. Cl.: C12N 9/12, C12Q 1/48, C07C 15/00

(54) **High resolution complex structure and allosteric effects of low molecular weight activators on the protein kinase PDK1**

(71) Applicant: Universität des Saarlandes, 66123 Saarbrücken (DE)
(72) Inventor: Biondi, Ricardo M., 66424 Homburg (DE); Hindie, Valerie, 75015 Paris (FR)
(74) Representative: von Kreisler Selting Werner

(57) **Abstract**

The present invention provides a mutant form of a protein kinase its production and its use for identifying compounds that bind to the PIF-binding pocket allosteric site of the protein kinase.

SEQUENCE LISTING

<110>  Universitaet des Saarlandes

<120>  HIGH RESOLUTION COMPLEX STRUCTURE AND ALLOSTERIC EFFECTS OF LOW
       MOLECULAR WEIGHT ACTIVATORS ON THE PROTEIN KINASE PDK1

<130>  081894ep

<160>  12

<170>  PatentIn version 3.3

<210>  1
<211>  556
<212>  PRT
<213>  Homo sapiens

<400>  1

Met Ala Arg Thr Thr Ser Gln Leu Tyr Asp Ala Val Pro Ile Gln Ser
1               5                   10                  15

Ser Val Val Leu Cys Ser Cys Pro Ser Pro Ser Met Val Arg Thr Gln
            20                  25                  30

Thr Glu Ser Ser Thr Pro Pro Gly Ile Pro Gly Gly Ser Arg Gln Gly
        35                  40                  45

Pro Ala Met Asp Gly Thr Ala Ala Glu Pro Arg Pro Gly Ala Gly Ser
    50                  55                  60

Leu Gln His Ala Gln Pro Pro Pro Gln Pro Arg Lys Lys Arg Pro Glu
65                  70                  75                  80

Asp Phe Lys Phe Gly Lys Ile Leu Gly Glu Gly Ser Phe Ser Thr Val
                85                  90                  95

Val Leu Ala Arg Glu Leu Ala Thr Ser Arg Glu Tyr Ala Ile Lys Ile
            100                 105                 110

Leu Glu Lys Arg His Ile Ile Lys Glu Asn Lys Val Pro Tyr Val Thr
        115                 120                 125

Arg Glu Arg Asp Val Met Ser Arg Leu Asp His Pro Phe Phe Val Lys
    130                 135                 140

Leu Tyr Phe Thr Phe Gln Asp Asp Glu Lys Leu Tyr Phe Gly Leu Ser
145                 150                 155                 160

Tyr Ala Lys Asn Gly Glu Leu Leu Lys Tyr Ile Arg Lys Ile Gly Ser
                165                 170                 175

Phe Asp Glu Thr Cys Thr Arg Phe Tyr Thr Ala Glu Ile Val Ser Ala
            180                 185                 190

Leu Glu Tyr Leu His Gly Lys Gly Ile Ile His Arg Asp Leu Lys Pro
        195                 200                 205

Glu Asn Ile Leu Leu Asn Glu Asp Met His Ile Gln Ile Thr Asp Phe
    210                 215                 220

Gly Thr Ala Lys Val Leu Ser Pro Glu Ser Lys Gln Ala Arg Ala Asn
225                 230                 235                 240

Ser Phe Val Gly Thr Ala Gln Tyr Val Ser Pro Glu Leu Leu Thr Glu
                245                 250                 255

Lys Ser Ala Cys Lys Ser Ser Asp Leu Trp Ala Leu Gly Cys Ile Ile
            260                 265                 270

Tyr Gln Leu Val Ala Gly Leu Pro Pro Phe Arg Ala Gly Asn Glu Tyr
        275                 280                 285

Leu Ile Phe Gln Lys Ile Ile Lys Leu Glu Tyr Asp Phe Pro Glu Lys
    290                 295                 300

Phe Phe Pro Lys Ala Arg Asp Leu Val Glu Lys Leu Leu Val Leu Asp
305                 310                 315                 320

Ala Thr Lys Arg Leu Gly Cys Glu Glu Met Glu Gly Tyr Gly Pro Leu
                325                 330                 335

Lys Ala His Pro Phe Phe Glu Ser Val Thr Trp Glu Asn Leu His Gln
            340                 345                 350

Gln Thr Pro Pro Lys Leu Thr Ala Tyr Leu Pro Ala Met Ser Glu Asp
        355                 360                 365

Asp Glu Asp Cys Tyr Gly Asn Tyr Asp Asn Leu Leu Ser Gln Phe Gly
    370                 375                 380

Cys Met Gln Val Ser Ser Ser Ser Ser Ser His Ser Leu Ser Ala Ser
385                 390                 395                 400

Asp Thr Gly Leu Pro Gln Arg Ser Gly Ser Asn Ile Glu Gln Tyr Ile
                405                 410                 415

His Asp Leu Asp Ser Asn Ser Phe Glu Leu Asp Leu Gln Phe Ser Glu
            420                 425                 430

Asp Glu Lys Arg Leu Leu Leu Glu Lys Gln Ala Gly Gly Asn Pro Trp
        435                 440                 445

His Gln Phe Val Glu Asn Asn Leu Ile Leu Lys Met Gly Pro Val Asp
    450                 455                 460

Lys Arg Lys Gly Leu Phe Ala Arg Arg Arg Gln Leu Leu Leu Thr Glu
465                 470                 475                 480

Gly Pro His Leu Tyr Tyr Val Asp Pro Val Asn Lys Val Leu Lys Gly
                485                 490                 495

Glu Ile Pro Trp Ser Gln Glu Leu Arg Pro Glu Ala Lys Asn Phe Lys
            500                 505                 510

Thr Phe Phe Val His Thr Pro Asn Arg Thr Tyr Tyr Leu Met Asp Pro
        515                 520                 525

Ser Gly Asn Ala His Lys Trp Cys Arg Lys Ile Gln Glu Val Trp Arg
    530                 535                 540

Gln Arg Tyr Gln Ser His Pro Asp Ala Ala Val Gln
545                 550                 555

<210>  2
<211>  10
<212>  PRT
<213>  Homo sapiens

<400>  2

Ala Gly Asn Glu Tyr Leu Ile Phe Gln Lys
1               5                   10

<210>  3
<211>  8
<212>  PRT
<213>  Homo sapiens

<400>  3

Leu Asp His Pro Phe Phe Val Lys
1               5

<210>  4
<211>  312
<212>  PRT
<213>  Artificial

<220>
<223>  modified human PDK1

<400>  4

Gly Ala Met Asp Gly Thr Ala Ala Glu Pro Arg Pro Gly Ala Gly Ser
1               5                   10                  15

Leu Gln His Ala Gln Pro Pro Pro Gln Pro Arg Lys Lys Arg Pro Glu
            20                  25                  30

Asp Phe Lys Phe Gly Lys Ile Leu Gly Glu Gly Ser Phe Ser Thr Val
        35                  40                  45

Val Leu Ala Arg Glu Leu Ala Thr Ser Arg Glu Tyr Ala Ile Lys Ile
    50                  55                  60

Leu Glu Lys Arg His Ile Ile Lys Glu Asn Lys Val Pro Tyr Val Thr
65                  70                  75                  80

Arg Glu Arg Asp Val Met Ser Arg Leu Asp His Pro Phe Phe Val Lys
                85                  90                  95

Leu Tyr Phe Thr Phe Gln Asp Asp Glu Lys Leu Tyr Phe Gly Leu Ser
            100                 105                 110

Tyr Ala Lys Asn Gly Glu Leu Leu Lys Tyr Ile Arg Lys Ile Gly Ser
        115                 120                 125

Phe Asp Glu Thr Cys Thr Arg Phe Tyr Thr Ala Glu Ile Val Ser Ala
    130                 135                 140

Leu Glu Tyr Leu His Gly Lys Gly Ile Ile His Arg Asp Leu Lys Pro
145                 150                 155                 160

Glu Asn Ile Leu Leu Asn Glu Asp Met His Ile Gln Ile Thr Asp Phe
                165                 170                 175

Gly Thr Ala Lys Val Leu Ser Pro Glu Ser Lys Gln Ala Arg Ala Asn
            180                 185                 190

Ser Phe Val Gly Thr Ala Gln Tyr Val Ser Pro Glu Leu Leu Thr Glu
        195                 200                 205

Lys Ser Ala Cys Lys Ser Ser Asp Leu Trp Ala Leu Gly Cys Ile Ile
    210                 215                 220

Tyr Gln Leu Val Ala Gly Leu Pro Pro Phe Arg Ala Gly Asn Glu Gly
225                 230                 235                 240

Leu Ile Phe Ala Lys Ile Ile Lys Leu Glu Tyr Asp Phe Pro Glu Lys
                245                 250                 255

Phe Phe Pro Lys Ala Arg Asp Leu Val Glu Lys Leu Leu Val Leu Asp
            260                 265                 270

Ala Thr Lys Arg Leu Gly Cys Glu Glu Met Glu Gly Tyr Gly Pro Leu
        275                 280                 285

Lys Ala His Pro Phe Phe Glu Ser Val Thr Trp Glu Asn Leu His Gln
    290                 295                 300

Gln Thr Pro Pro Lys Leu Thr Ala
305                 310

<210>  5
<211>  293
<212>  PRT
<213>  Artificial

<220>
<223>  modified human PDK1

<400>  5

Ala Gln Pro Pro Pro Gln Pro Arg Lys Lys Arg Pro Glu Asp Phe Lys
1               5                   10                  15

Phe Gly Lys Ile Leu Gly Glu Gly Ser Phe Ser Thr Val Val Leu Ala
            20                  25                  30

Arg Glu Leu Ala Thr Ser Arg Glu Tyr Ala Ile Lys Ile Leu Glu Lys
        35                  40                  45

Arg His Ile Ile Lys Glu Asn Lys Val Pro Tyr Val Thr Arg Glu Arg
    50                  55                  60

Asp Val Met Ser Arg Leu Asp His Pro Phe Phe Val Lys Leu Tyr Phe
65                  70                  75                  80

Thr Phe Gln Asp Asp Glu Lys Leu Tyr Phe Gly Leu Ser Tyr Ala Lys
                85                  90                  95

Asn Gly Glu Leu Leu Lys Tyr Ile Arg Lys Ile Gly Ser Phe Asp Glu
            100                 105                 110

Thr Cys Thr Arg Phe Tyr Thr Ala Glu Ile Val Ser Ala Leu Glu Tyr
        115                 120                 125

Leu His Gly Lys Gly Ile Ile His Arg Asp Leu Lys Pro Glu Asn Ile

130                 135                 140

Leu Leu Asn Glu Asp Met His Ile Gln Ile Thr Asp Phe Gly Thr Ala
145                 150                 155                 160

Lys Val Leu Ser Pro Glu Ser Lys Gln Ala Arg Ala Asn Ser Phe Val
                165                 170                 175

Gly Thr Ala Gln Tyr Val Ser Pro Glu Leu Leu Thr Glu Lys Ser Ala
            180                 185                 190

Cys Lys Ser Ser Asp Leu Trp Ala Leu Gly Cys Ile Ile Tyr Gln Leu
        195                 200                 205

Val Ala Gly Leu Pro Pro Phe Arg Ala Gly Asn Glu Gly Leu Ile Phe
    210                 215                 220

Ala Lys Ile Ile Lys Leu Glu Tyr Asp Phe Pro Glu Lys Phe Phe Pro
225                 230                 235                 240

Lys Ala Arg Asp Leu Val Glu Lys Leu Leu Val Leu Asp Ala Thr Lys
                245                 250                 255

Arg Leu Gly Cys Glu Glu Met Glu Gly Tyr Gly Pro Leu Lys Ala His
            260                 265                 270

Pro Phe Phe Glu Ser Val Thr Trp Glu Asn Leu His Gln Gln Thr Pro
        275                 280                 285

Pro Lys Leu Thr Ala
    290

<210>  6
<211>  332
<212>  PRT
<213>  Artificial

<220>
<223>  modified human PDK1

<400>  6

His His His His His His Asp Tyr Asp Ile Pro Thr Thr Glu Asn Leu
1               5                   10                  15

Tyr Phe Gln Gly Gly Ala Met Asp Gly Thr Ala Ala Glu Pro Arg Pro
            20                  25                  30

Gly Ala Gly Ser Leu Gln His Ala Gln Pro Pro Pro Gln Pro Arg Lys
        35                  40                  45

Lys Arg Pro Glu Asp Phe Lys Phe Gly Lys Ile Leu Gly Glu Gly Ser
    50                  55                  60

Phe Ser Thr Val Val Leu Ala Arg Glu Leu Ala Thr Ser Arg Glu Tyr
65                  70                  75                  80

Ala Ile Lys Ile Leu Glu Lys Arg His Ile Ile Lys Glu Asn Lys Val
                85                  90                  95

Pro Tyr Val Thr Arg Glu Arg Asp Val Met Ser Arg Leu Asp His Pro
            100                 105                 110

Phe Phe Val Lys Leu Tyr Phe Thr Phe Gln Asp Asp Glu Lys Leu Tyr
        115                 120                 125

Phe Gly Leu Ser Tyr Ala Lys Asn Gly Glu Leu Leu Lys Tyr Ile Arg
    130                 135                 140

Lys Ile Gly Ser Phe Asp Glu Thr Cys Thr Arg Phe Tyr Thr Ala Glu
145                 150                 155                 160

Ile Val Ser Ala Leu Glu Tyr Leu His Gly Lys Gly Ile Ile His Arg
                165                 170                 175

Asp Leu Lys Pro Glu Asn Ile Leu Leu Asn Glu Asp Met His Ile Gln
            180                 185                 190

Ile Thr Asp Phe Gly Thr Ala Lys Val Leu Ser Pro Glu Ser Lys Gln
        195                 200                 205

Ala Arg Ala Asn Ser Phe Val Gly Thr Ala Gln Tyr Val Ser Pro Glu
    210                 215                 220

Leu Leu Thr Glu Lys Ser Ala Cys Lys Ser Ser Asp Leu Trp Ala Leu
225                 230                 235                 240

Gly Cys Ile Ile Tyr Gln Leu Val Ala Gly Leu Pro Pro Phe Arg Ala
                245                 250                 255

Gly Asn Glu Gly Leu Ile Phe Ala Lys Ile Ile Lys Leu Glu Tyr Asp
            260                 265                 270

Phe Pro Glu Lys Phe Phe Pro Lys Ala Arg Asp Leu Val Glu Lys Leu
        275                 280                 285

Leu Val Leu Asp Ala Thr Lys Arg Leu Gly Cys Glu Glu Met Glu Gly

290                 295                 300

Tyr Gly Pro Leu Lys Ala His Pro Phe Phe Glu Ser Val Thr Trp Glu
305                 310                 315                 320

Asn Leu His Gln Gln Thr Pro Pro Lys Leu Thr Ala
                325                 330

<210>  7
<211>  312
<212>  PRT
<213>  Artificial

<220>
<223>  modified human PDK1

<400>  7

Gly Pro Met Asp Gly Thr Ala Ala Glu Pro Arg Pro Gly Ala Gly Ser
1               5                   10                  15

Leu Gln His Ala Gln Pro Pro Pro Gln Pro Arg Lys Lys Arg Pro Glu
            20                  25                  30

Asp Phe Lys Phe Gly Lys Ile Leu Gly Glu Gly Ser Phe Ser Thr Val
        35                  40                  45

Val Leu Ala Arg Glu Leu Ala Thr Ser Arg Glu Tyr Ala Ile Lys Ile
    50                  55                  60

Leu Glu Lys Arg His Ile Ile Lys Glu Asn Lys Val Pro Tyr Val Thr
65                  70                  75                  80

Arg Glu Arg Asp Val Met Ser Arg Leu Asp His Pro Phe Phe Val Lys
                85                  90                  95

Leu Tyr Phe Thr Phe Gln Asp Asp Glu Lys Leu Tyr Phe Gly Leu Ser
            100                 105                 110

Tyr Ala Lys Asn Gly Glu Leu Leu Lys Tyr Ile Arg Lys Ile Gly Ser
        115                 120                 125

Phe Asp Glu Thr Cys Thr Arg Phe Tyr Thr Ala Glu Ile Val Ser Ala
    130                 135                 140

Leu Glu Tyr Leu His Gly Lys Gly Ile Ile His Arg Asp Leu Lys Pro
145                 150                 155                 160

Glu Asn Ile Leu Leu Asn Glu Asp Met His Ile Gln Ile Thr Asp Phe
                165                 170                 175

Gly Thr Ala Lys Val Leu Ser Pro Glu Ser Lys Gln Ala Arg Ala Asn
            180                 185                 190

Ser Phe Val Gly Thr Ala Gln Tyr Val Ser Pro Glu Leu Leu Thr Glu
        195                 200                 205

Lys Ser Ala Cys Lys Ser Ser Asp Leu Trp Ala Leu Gly Cys Ile Ile
    210                 215                 220

Tyr Gln Leu Val Ala Gly Leu Pro Pro Phe Arg Ala Gly Asn Glu Tyr
225                 230                 235                 240

Leu Ile Phe Gln Lys Ile Ile Lys Leu Glu Tyr Asp Phe Pro Glu Lys
                245                 250                 255

Phe Phe Pro Lys Ala Arg Asp Leu Val Glu Lys Leu Leu Val Leu Asp
            260                 265                 270

Ala Thr Lys Arg Leu Gly Cys Glu Glu Met Glu Gly Tyr Gly Pro Leu
        275                 280                 285

Lys Ala His Pro Phe Phe Glu Ser Val Thr Trp Glu Asn Leu His Gln
    290                 295                 300

Gln Thr Pro Pro Lys Leu Thr Ala
305                 310


<210>  8
<211>  16
<212>  PRT
<213>  Artificial

<220>
<223>  PDK1 substrate

<400>  8

Lys Thr Phe Cys Gly Thr Pro Glu Tyr Leu Ala Pro Glu Val Arg Arg
1               5                   10                  15


<210>  9
<211>  24
<212>  PRT
<213>  Artificial

<220>
<223>  PDK1 substrate

<400>  9

Arg Glu Pro Arg Ile Leu Ser Glu Glu Glu Gln Glu Met Phe Arg Asp

1               5                   10                  15

Phe Asp Tyr Ile Ala Asp Trp Cys
            20

<210>  10
<211>  33
<212>  PRT
<213>  Artificial

<220>
<223>  PDK1 substrate

<400>  10

Lys Gln Thr Pro Val Asp Ser Pro Pro Asp Asp Ser Thr Leu Ser Glu
1               5                   10                  15

Ser Ala Asn Gln Val Phe Leu Gly Phe Thr Pro Tyr Val Ala Pro Ser
            20                  25                  30

Val

<210>  11
<211>  861
<212>  PRT
<213>  Candida albicans

<400>  11

Met Ser Gly Leu Glu His Leu Gln Asn Leu Ser Ile Gln Pro Ser Asn
1               5                   10                  15

Arg Ser Thr Gln Gln Gln Gln Thr Asn Ser Gln Pro Gln Ser Gln Tyr
            20                  25                  30

Ser Gln Ser Ala Ala Lys Arg Arg Ser Ala Arg Asp Tyr Gln Phe Gly
        35                  40                  45

Ala Lys Ile Gly Glu Gly Ser Tyr Ser Thr Val Tyr Ser Ala Leu Asp
    50                  55                  60

Lys Tyr Thr Asn Arg Thr Tyr Ala Ile Lys Val Leu Ser Lys Arg His
65                  70                  75                  80

Ile Val Lys Glu Asn Lys Ile Lys Tyr Val Asn Ile Glu Lys Thr Thr
                85                  90                  95

Leu Asn Arg Leu Gly Gln Gln His Pro Gly Ile Val Gln Leu Tyr Tyr
            100                 105                 110

Thr Phe Gln Asp Glu Ser Ser Leu Phe Phe Val Leu Asp Phe Ala Glu
        115                 120                 125

Tyr Gly Glu Leu Leu Ser Ile Ile Arg Lys Phe Gly Ser Leu Ser Glu
    130                 135                 140

Ser Val Ser Lys Phe Tyr Met Cys Gln Ile Ile Asp Ala Val Arg Phe
145                 150                 155                 160

Ile His Ser Lys Gly Val Ile His Arg Asp Leu Lys Pro Glu Asn Ile
                165                 170                 175

Leu Val Gly His Asp Phe Asn Leu Lys Ile Thr Asp Phe Gly Ala Ala
            180                 185                 190

Lys Leu Leu Gly Asn His Glu Glu Phe Asn Gly Glu Lys Ile Asp Tyr
        195                 200                 205

Asn Ser Val Asp Ala Ser Pro Glu Val Ser Asp Thr Asp Arg Lys Gly
    210                 215                 220

Ser Phe Val Gly Thr Ala Glu Tyr Val Ser Pro Glu Leu Leu Lys His
225                 230                 235                 240

Asn Ile Cys Gly Phe Glu Ser Asp Ile Trp Ala Ile Gly Cys Ile Leu
                245                 250                 255

Tyr Gln Phe Phe Asn Gly Val Pro Pro Phe Lys Gly Ser Thr Glu Tyr
            260                 265                 270

Leu Thr Phe Glu Lys Ile Ile Asn Val Asp Tyr Ser Tyr Lys Ser Pro
        275                 280                 285

Ile Pro Leu Pro Pro Asp Val Ile Gln Leu Ile Asp Ser Ile Leu Ile
    290                 295                 300

Ser Glu Pro Ser Ala Arg Ala Thr Ile Pro His Ile Met Ser Ser Arg
305                 310                 315                 320

Trp Phe Ser Asp Ile Pro Trp Asp Asp Gln Asn Phe Ile Trp His Arg
                325                 330                 335

Lys Val Pro Arg Phe Glu Ala Phe Gly Ala Ile Thr Pro Ser Pro Ala
            340                 345                 350

Met Pro Pro Met Met Lys Asn Gly Thr Asn Arg Asn Val Asn Lys Ser
        355                 360                 365

Asn Ser Tyr His Gln Leu Gln Ser Gln Ile Ser Ala Ser Glu Phe Gly
    370                 375                 380

Phe Ile Pro Ser Ile Ala Ala Lys Lys Ser Tyr Lys Pro Pro Thr Met
385                 390                 395                 400

Ile Lys Asn Asn Val Ala Met Pro Ala Leu Asn Gln Thr Thr Tyr Asn
                405                 410                 415

His Asn Tyr Gln Gln His Val Pro Leu Pro Pro Pro Val Ser Ser Gln
            420                 425                 430

Pro Leu Thr Gln Gln Asn Val Pro Gln Gln Ile Gln Pro Gln Arg Ser
        435                 440                 445

Gly Ser Ala Gln Thr Pro Gln Phe Ala Pro Val Ser Ala Ser Ser Arg
    450                 455                 460

Gln Pro Val Gly Asn Met Gln Thr Arg Gln Ala Ala Pro Gln Val Leu
465                 470                 475                 480

Val Gln Gln Lys Thr Pro Pro Phe Pro Lys Glu Lys Asn Ser Thr Pro
                485                 490                 495

Asn Leu Arg Asp Asn Thr Ser Phe Ala Gln Met Ala Pro Arg Arg Asn
            500                 505                 510

Gly Glu Ile Pro Gly Gln Pro Gln Gln Gln Ile Pro Leu Pro Lys Ser
        515                 520                 525

Gln Gln Ser Pro Gln Pro Gln Pro Lys Ala Gln Tyr Gln Ser Gln Gln
    530                 535                 540

Gln Lys Gln Ser Glu Asp Ile Arg Gly Ser Lys Asn Thr Ser Ala Asn
545                 550                 555                 560

Ala Ala Ala Ala Ala Phe Ala Thr Lys Asn Leu Pro Gln Pro Asn His
                565                 570                 575

Lys Thr Ser Lys Asp Ser Ser Thr Pro Pro Pro Pro Pro Pro Leu Pro
            580                 585                 590

Pro Lys Ile Lys Lys Val Gln Glu Asn Phe Ile Lys Phe Asn Glu Val
        595                 600                 605

Ser Gly Leu Leu Lys Pro Asp Glu Lys Ile Leu Lys Met Asp Asn Leu

610                 615                 620

Ile Lys Ser Val Leu Ser Lys Arg Tyr Leu Asn Lys Pro Glu Tyr Gln
625                 630                 635                 640

Val Leu Asp Asp Ser Val Leu Asp Glu Leu Thr Ser Lys Tyr Ala Ser
                645                 650                 655

Ile Leu Lys Gln Glu Ser Lys Ala Val Ile Thr Ile Val Thr Asn Met
            660                 665                 670

Ala Arg Val Phe Phe Ile Asp Ala Phe Leu Asn Val Met Leu Val Asp
        675                 680                 685

Leu Lys Ala Asn Lys Gly Ala Asp Tyr Ser Met Tyr Asp Tyr Glu Phe
    690                 695                 700

Glu Asn Thr Val Asp Glu Asp Gly Asn Leu Leu Ser Lys Gln Asp Glu
705                 710                 715                 720

Thr Phe Gly Tyr Leu Ile Ile Glu Leu Ile Lys Asp Gly Gly Asp Leu
                725                 730                 735

Val Phe Leu Gln Arg Val Ser Asp Glu Gln Arg Leu Arg Leu Thr Asn
            740                 745                 750

Ala Val Lys Val Leu Asp Lys Lys Gly Glu Glu Val Val Val Gly Ser
        755                 760                 765

Ser His Gly Trp Ile Asp Cys Leu Leu Ile Ala Arg Glu Ile Ser Ile
    770                 775                 780

Glu Gln Ser Glu Asp Asn Lys Lys Pro Arg Ser Val Ser Ser Ser Ser
785                 790                 795                 800

Asn Val Ser Ile Asn Glu Val Lys Ser Glu Lys Ala Lys Lys Lys Lys
                805                 810                 815

Ser Ser Leu Ser Asn Lys Lys Pro Arg Ser Lys Lys Lys Gly Thr Ser
            820                 825                 830

Ser Asn Gly Thr Pro Gln Thr Thr Pro Thr Pro Thr Gln His Ser Asn
        835                 840                 845

Phe Ala Phe Ala Ala Ala Ala Ala Val His His Gln Lys
    850                 855                 860

<210>  12
<211>  944
<212>  PRT
<213>  Candida albicans

<400>  12

Met His Lys Phe Arg Tyr Ser Leu His Gln His Tyr Ser Lys Arg Asn
1               5                   10                  15

Ser Ser Asp Lys Ser Lys Asp Ser Pro Ile Ser Gln Asn Ser Asn Glu
            20                  25                  30

Glu Asn Asp Ser Thr Lys Leu Ser Ser Ser Ser Leu Gln Asp Leu His
        35                  40                  45

Asp Asp Leu Asp Asp Ile Tyr Asn Asn Tyr Thr Leu Ala Gln Gly Thr
    50                  55                  60

Asn Asn Asn Ser Val Asp Thr Leu Asp Ser Glu Asn Asn Gln Ala Ile
65                  70                  75                  80

Asn Lys Phe Ile Asp Lys Pro Pro Ala Ile His Gly Met Glu Pro Gln
                85                  90                  95

Leu Pro Val Met His Val Ser Ser Arg Leu Ser Ser Leu Gly Asn Thr
            100                 105                 110

Thr Asn Glu Thr Gly Glu Ser Ile Ala Lys Ser Ala Pro Gly Thr Pro
        115                 120                 125

Leu Ser Ser His Ser Phe Asp Phe Arg Pro His His Pro Arg Ala Val
    130                 135                 140

Thr Asn Ser Ser Leu Asn Val Leu Leu Asp Thr Pro Asn Val Ser Ser
145                 150                 155                 160

Glu Phe Asn His Leu Val Asp Gln Thr Pro Pro Asn Glu Ser Val Glu
                165                 170                 175

Arg Phe Asp Asp Ser Asn Asn Thr Val Asp Asn Thr Glu Glu Glu Glu
            180                 185                 190

Asn Asn Asp Asp Thr Asp Glu Ile Pro Lys Ser Glu Thr Leu Lys Gln
        195                 200                 205

Asn Glu Glu Asn Trp Glu Lys Lys Gly Ala Ala Val Lys Thr Ile Lys
    210                 215                 220

Thr Met Asp Gly Glu Met Lys Thr Ile Arg Arg Asn Val Thr Asp Phe
225                 230                 235                 240

Lys Phe Gly Lys Glu Leu Gly Glu Gly Ser Tyr Ser Thr Val Ile Leu
                245                 250                 255

Ala Thr Asp Lys Ile Thr Gly Lys Gln Tyr Ala Val Lys Val Leu Asp
            260                 265                 270

Lys Arg His Ile Ile Lys Glu Lys Lys Val Lys Tyr Val Asn Ile Glu
        275                 280                 285

Lys His Ala Leu Asn Arg Leu Ser Asn Arg Leu Gly Val Ile Ser Leu
    290                 295                 300

Tyr Phe Thr Phe Gln Asp Lys Asp Ser Leu Tyr Phe Val Leu Asp Tyr
305                 310                 315                 320

Ala Ser Asn Gly Glu Leu Leu Thr Leu Ile Lys Arg Tyr Asn Thr Leu
                325                 330                 335

Asn Glu Glu Cys Thr Arg His Phe Gly Ala Gln Ile Leu Asp Ala Ile
            340                 345                 350

Lys Tyr Met His Asp Asn Gly Val Ile His Arg Asp Leu Lys Pro Glu
        355                 360                 365

Asn Ile Leu Leu Asp Asp Lys Met Arg Ile Gln Ile Thr Asp Phe Gly
    370                 375                 380

Thr Ala Arg Leu Leu Glu Lys Lys Asn Asp Glu Ser Glu Glu Tyr Pro
385                 390                 395                 400

Val Asp Val Arg Ala Lys Ser Phe Val Gly Thr Ala Glu Tyr Val Ser
                405                 410                 415

Pro Glu Leu Leu Glu Asn Lys Tyr Cys Gly Lys Pro Gly Asp Val Trp
            420                 425                 430

Ala Phe Gly Cys Ile Ile Tyr Gln Met Ile Ala Gly Lys Pro Pro Phe
        435                 440                 445

Lys Ala Thr Asn Glu Tyr Leu Thr Phe Gln Lys Ile Thr Lys Leu Gln
    450                 455                 460

Phe Ala Phe Ser Ala Gly Phe Pro Thr Ile Ile Arg Asp Leu Ile Lys
465                 470                 475                 480

Lys Ile Leu Val Leu Gln Pro Ser Arg Arg Ala Thr Ile Pro Glu Ile
                485                 490                 495

Gln Lys His Tyr Phe Phe Gln Ser Val Asp Phe Lys Asp Phe Asp Ser
            500                 505                 510

Ile Trp Leu Ser Asp Pro Pro Glu Ile Gly Pro Tyr Lys Met Thr Ala
        515                 520                 525

Lys Ser Met Met Lys Val Pro Glu Leu Asn Lys Ala Pro Ile Thr Thr
    530                 535                 540

Val Ile Lys Lys Asn Val Lys Lys Ser Thr Asn Ser Asn Ser Asn Thr
545                 550                 555                 560

Asn Asn Val Ala Thr Ala Val Gly Gly Ser Ser Ser Asn Gly His Lys
                565                 570                 575

Gly Ser Ser Pro Thr Pro Glu Lys Glu Pro Ser Pro Ala Thr Ile Asn
            580                 585                 590

Asn Lys Ser Thr Glu Lys Val Ser Ala Ala Ser Val Ala Ala Tyr Val
        595                 600                 605

Leu Asn Lys Pro Ala Thr Asn Gln Asn Ser Ser Thr Ser Glu Asp Ser
    610                 615                 620

Ser Lys Arg Ser Ser Asn Ser Asn Glu Thr Arg Lys Leu Ser Tyr Ser
625                 630                 635                 640

Gln Gln Asp Tyr Ile Pro Gly Thr Asn Ile Leu Arg Pro Gln Ile Ser
                645                 650                 655

Thr Arg Pro Ser Val Gly Ser Tyr Val Lys Thr Thr Pro Ser Lys Asp
            660                 665                 670

Arg Lys Thr Leu Thr Lys Val Pro Ser Asn Ile His Gln Gln Gln Glu
        675                 680                 685

Lys Val Lys Pro Lys Val Met Glu Val Lys Pro Ala Thr Thr Leu Glu
    690                 695                 700

Ala Ala Trp Glu Pro Tyr Leu Thr His Pro Asp Glu Arg Ile Leu Arg
705                 710                 715                 720

Ile Gly Pro Val Ile Ala His Lys Glu Pro Thr Glu Pro Phe Glu Lys
                725                 730                 735

Lys Asn Lys Ala Ser Leu His Ile Ser Pro Leu Asp Ile Asn Lys Glu
            740                 745                 750

Gln Arg Ser Arg Ser Asn Thr Ser Leu Leu Thr Gln Ile Val Asn Glu
        755                 760                 765

Val Asn Asn Asn Thr Ser Glu Leu Lys Lys Val Glu Asn Ala Asp Glu
    770                 775                 780

Ser Leu Ala Ile Ile Glu Pro Gln Tyr Asn Met Lys Arg Ser Pro Thr
785                 790                 795                 800

Ser Asp Ser Lys Lys Ser Met Asp Ile Glu Arg Ser Ala Ser Thr Ser
                805                 810                 815

Gly Ser Arg Ile Ser Lys Lys Ala Ile Phe Lys Lys Leu Gly Phe Ser
            820                 825                 830

His Leu Glu Lys Asn Asp Ser Glu Glu Ser Asn Gly Pro Ser Leu Thr
        835                 840                 845

Glu Lys Pro Gln Thr Cys Thr Leu Val Val Thr Thr His Gly Arg Ala
    850                 855                 860

Leu Leu Phe Ile Arg Asn Asp Ile Glu Ser Asn Tyr Leu Leu Ile Ala
865                 870                 875                 880

Glu Ile Lys Leu Lys Tyr Pro Phe Ile His Phe Gln Glu Leu Val Ile
                885                 890                 895

Ser Gln Thr Lys Phe Ser Lys Leu Val Pro Ser Val Gly Val Phe Val
            900                 905                 910

Ile Ser Ser Ile Asp Asn Ser Leu Ile Phe Glu Val Glu Lys Phe Glu
        915                 920                 925

Val Asn Gln Trp Thr Glu Ala Leu Ala Lys Ser Lys Tyr Asn Glu Ile
    930                 935                 940

## Description

The present invention provides a mutant form of a protein kinase its production and its use for identifying compounds that bind to the PIF-binding pocket allosteric site of the protein kinase.

### Background of the Invention

The complexity of cells and organisms require that physiological functions have to be regulated. The most studied mechanism of regulation of cellular activities is protein phosphorylation. Recent proteomic studies suggest that a large proportion of cellular proteins are phosphorylated and that stimulation of cells by one growth factor modulates the phosophorylation state of 14% cellular phosphorylated sites (Olsen, J. V. et al., Cell 127:635-648 (2006)).

The covalent binding of a phosphate to a protein can modulate signalling events by modulating phosphorylation-dependent protein-protein interactions and also by prompting conformational changes on the phosphorylated protein or on the interacting proteins (Johnson, L. N. and Lewis, R. J., Chem. Rev. 101:2209-2242 (2001); Pawson, T. and Scott, J. D., Trends Biochem. Sci. 30:286-290 (2005)). Conformational changes in proteins can easily be followed on enzymes by measurement of the enzymatic activity and numerous enzymes are known to be activated or inhibited by protein phosphorylation. In spite of the wide existence of protein phosphorylation in nature, the molecular events that trigger phosphorylation-dependent conformational changes remain vastly unknown.

The enzymes that catalyse protein phosphorylation, the protein kinases, are themselves often tightly regulated by phosphorylation (Huse, M. and Kuriyan, J. Cell 109:275-282 (2002)). Work over the last 8 years has shown that the AGC kinase group share a core mechanism of regulation mediated by intra-molecular docking of a phosphorylated C-terminal hydrophobic motif (HM) to a hydrophobic pocket and associated phosphate binding site on the small lobe of the kinase domain (termed the HM/PIF-binding pocket), located between the α-C-helix, α-B-helix, β-4 and β-5 sheets (Pearl, L. H. and Barford, D., Curr. Opin. Struct. Biol. 12:761-767 (2002); Biondi, R. M. and Nebreda, A. R., Biochem. J. 372:1-13 (2003); Newton, A. C., Chem. Rev. 101:2353-2364 (2001)). Docking of the phosphorylated HM to the HM/PIF-binding pocket and its associated phosphate binding site activates AGC kinases from different families, e.g. PDK1 (Biondi, R. M. et al., Embo. J. 19:979-988 (2000); Frodin, M. et al., Embo. J. 19:2924-2934 (2000)), PKB (also termed Akt) (Yang, J. et al., Nat. Struct. Biol. 9:940-944 (2002); Yang, J. et al., Mol. Cell 9:1227-1240 (2002); Frodin, M. et al., Embo. J. 21:5396-5407 (2002)), MSK, RSK, SGK and S6K (Frodin, M. et al., Embo. J. 21:5396-5407 (2002)), and an analogous mechanism is also conserved in Aurora family of kinases (Bayliss, R. et al., Mol. Cell 12:851-862 (2003)) that are related but not considered AGC kinases (Manning, G. et al., Science 298:1912-1934 (2002)). In addition, another C-terminal phosphorylation, termed "turn-motif" or "zipper" (Z) phosphorylation site, originally described in classical PKCs (Newton, A. C., Biochem. J. 370:361-371 (2003); Parker, P. J. and Parkinson, S. J., Biochem. Soc. Trans. 29:860-863 (2001)), also stabilizes the active conformation of AGC kinases by binding to a second phosphate binding site on the small lobe of kinases, acting like a zipper and prompting the binding of the hydrophobic motif to its HM/PIF-pocket binding site (Hauge, C. et al., Embo. J. 26:2251-2261 (2007)). Interestingly, the AGC protein kinase PDK1, that is the upstream kinase for diverse AGC kinases, docks the phosphorylated HM of a subset of substrates in *trans.* The binding of the HM of substrates to the HM/PIF-binding pocket on PDK1 provides not only a docking interaction but also activates PDK1, which then phosphorylates the activation loop of substrates (Biondi, R. M., Trends Biochem. Sci. 29:136-142 (2004); Newton, A. C., Biochem. J. 370:361-371 (2003)). Thus, the current model suggests that upon PDK1 phosphorylation of the activation loop of substrates, all three phosphorylation sites stabilize the active conformation of AGC kinases intramolecularly, hiding the HM from PDK1. Altogether, we concluded that the key regulatory site in AGC kinases was the HM/PIF-pocket binding site on the catalytic domain, which is occupied intra-molecularly by the C-terminal HM in most kinases, and, in PDK1, it is a docking site that is required for the interaction with all substrates studied except PKB (Biondi, R. M. et al., Embo. J. 20:4380-4390 (2001); Collins, B. J. et al., Embo. J. 22:4202-4211 (2003)).

The crystal structures of AGC kinases PKA (Knighton, D. R. et al., Science 253:407-414 (1991)), PDK1 (Biondi, R. M. et al., Embo. J. 21:4219-4228 (2002)), PKB (Yang, J. et al., Nat. Struct. Biol. 9:940-944 (2002); Yang, J. et al., Mol. Cell 9:1227-1240 (2002); Huang, X. et al., Structure (Camb) 11:21-30 (2003)), MSK1 (Smith, K. J. et al., Structure (Camb) 12:1067-1077 (2004)) and ROCK (Jacobs, M., J. Biol. Chem. 281:260-268 (2006)) show a conserved catalytic core. Except for PDK1, all AGC kinase active structures have a HM bound to the HM/PIF-binding pocket. PDK1 crystal structure, on the other hand, has the pocket occupied by a Tyr residue from a neighbouring molecule along the crystal packing. In addition, PKB and MSK1 crystal structures have been solved in an inactive conformation in which the small lobe is disrupted and therefore lacks the HM/PIF binding pocket. In PKB, the inactive form is characterized by a nearly full disorder of αB- and αC-helices. In contrast, MSK1 inactive structure displays a newly formed three-stranded β-sheet in the place of the αB- and αC-helices.

An inactive PDK1 protein, mutated at the activation loop phosphorylation site (PDK1[Ser241Ala]) has also been crystallized (Komander, D. et al., J. Biol. Chem. 280:18797-18802 (2005)). However, this protein crystallized in the same crystal packing and had an identical conformation as the wild type active counterpart. The presence of a strong electron density in the place of the Ser241 phosphate suggested the presence of a molecule that partly mimicked the Ser241 phosphate function since it linked Arg131 on the α-C-helix with Arg204 in the catalytic loop from the large lobe. At any rate, it is not clear if the inactive structures observed in the crystals correspond to any inactive structure present in solution.

Protein phosphorylation transduces a large set of intracellular signals. One mechanism by which phosphorylation mediates signal transduction is by prompting conformational changes in the target protein or interacting proteins. Protein kinases are themselves regulated by phosphorylation. A novel crystal packing of PDK1 and the crystal structure of PDK1 bound to a compound activator was now found. In addition, the conformational change induced by activating compounds in solution was followed by monitoring the changes in fluorescence intensity of an ATP analogue and by performing deuterium exchange experiments. The results indicate that the binding of the small compound produces local changes at the target site, the PIF-binding pocket, and also changes at the ATP binding site. Altogether, we present molecular details of the allosteric changes induced by small compounds that trigger the activation of PDK1, mimicking phosphorylation-dependent conformational changes.

Based on the overall model of the mechanism of regulation of AGC kinases mediated by the HM/PIF-binding pocket, the site on AGC kinases was recently targeted and low molecular weight compounds activators of PDK1 were developed (Engel, M., Embo. J. 25:5469-5480 (2006)). These molecules prompted similar effects on activity as phosphorylated HM polypeptides, suggesting that it may be possible to rationally develop drugs to mimic phosphorylation dependent conformational changes in relevant proteins. However, the molecular details of the compound binding to PDK1 and the conformational changes involved in the activation mechanism remained unknown.

WO2003/104481 refers to for polypeptides derived from PDK1, for crystallography work and derived drug development. It was now found that the example polypeptide only crystallizes in only one crystal packing, which is not useful for the drug development to the PIF-pocket allosteric site.

WO 01/44497 discloses methods for identifying compounds that modulate the activity of PDK1-like kinases.

In addition, the PDK1 DNA and protein sequences are part of WO 98/41638 which relates to a substantially pure 3-phosphoinositide-dependent protein kinase that phosphorylates PKB wherein the protein kinase comprises two or more of three stated polypeptides (with up to 4 conservative substitutions). One of these polypeptides is AGNEYLIFQK (SEQ ID NO:2), another is LDHPFFVK SEQ ID NO:3).

However, it is of general interest to develop drugs targeting the protein kinase PDK1. The drugs can be directed to the ATP binding site (standard) or to allosteric sites, different from the ATP binding site. There is growing interest in the development of "allosteric" compounds to protein kinases. The PIF-binding pocket allosteric site is a possible drug target to regulate the conformation of AGC kinases and other kinases possessing a similar regulatory site. The PIF-binding pocket in PDK1 protein is a central regulatory site and recent work has shown that low molecular weight compounds developed against the PIF-binding pocket in AGC kinases can activate PDK1 in vitro and act as inhibitors of PDK1 signalling in compound treated cells.

Crystallography is of help in drug development since it is possible to obtain a 3D information, at molecular and atomic resolution, on the drug binding-site. With this information in hand, it is possible to perform in silico screenings of compound libraries, selecting those compounds predicted to bind to the site of interest. In addition, when the crystallographic information is obtained in the presence of a compound, the knowledge on the molecular details of the interaction is of great help in the rational drug design strategies, where compounds are not only developed based on structure-activity relationship of related compounds, but most importantly, on the actual mode of binding of compounds to the target site. Using crystallographic information on the drug target site helps drug developing increasing output, increasing speed of drug development.

Even if crystal structure information is of great importance in drug development, it should be noted that the crystal packing can influence the determined 3D derived structure. In such cases, the structure observed in the crystal may not reflect the exact conformation of the protein and may hamper drug development. It is also important to note that, the crystal packing itself may hamper the drug development project, by abolishing the formation of compound-protein complexes. For example, the PDK1 crystal obtained with the human PDK1 protein 51-360 (SEQ ID NO:7) does not expose the PIF-pocket to the solvent, but it is occupied by a Tyr residue from a neighboring PDK1 subunit. The pocket seems to be too shallow for compounds to bind. Further mutation of the Tyr288 residue to Ala did not render a stable protein.

Thus, the state of the art is the production of a hPDK1 protein which crystallizes in only one crystal packing, which does not allow the formation of complexes with PIF-pocket directed compounds. Moreover, the crystal structure shows a shallow PIF-pocket due to Phe157 positioning, which does not fit with the data that shows that the PDK1 protein indeed binds PIFtide and small compounds which are expected to require the depth in the pocket, for efficient binding. In other words, the crystal structure derived from the hPDK1 sequence known in the art, the PIF-pocket was not in a conformation competent for compound binding.

### Short Description of the Invention

It was now found that particular mutant PDK1 proteins are able to crystallize in different packings and diffract to high resolution. Most importantly, we show that these novel crystal packings allow the binding of small compounds and polypeptides to the PIF-pocket on PDK1. In particular it was found that a polypeptide comprising hPDK1 50-359 sequence, crystallizes in crystal packing II when its sequence is altered to express a Gly residue instead of Tyr288 and Ala in place of Gln292. Moreover it was found that it is possible to obtain PDK1-small compound complexes in crystal packing II. Finally, it was found that the double mutated protein further crystallizes in crystal packings III and IV, and that a hydrophobic-motif polypeptide can be complexed to the PIF-pocket. The knowledge at the PIF-pocket site in different crystal packings, together with the verification of the validity of the findings in solution should further strengthen the use of the crystallography data, not only for the PIF-pocket developments, but also for the development of ATP binding site compounds.

Thus particular PDK1 mutants were identified, notably the 288Gly and 192Ala mutant PDK1 proteins that are useful for crystallography work, including in silico screenings, to improve compound potency and, in general, to improve the time it takes to develop a drug targeting PDK1.

The protein mutant of the invention can be used for selecting or designing a compound for modulating the activity of PDK1. For example, using the mutant protein within a crystal and its derived crystal structure information it is possible to select or design a compound using molecular modeling means. In such strategies, the three dimentional structure of the mutant PDK1 is compared alternatively or simultaneously with the three-dimentional structure of one or more compounds and a compound that is predicted to interact with the said PDK1 mutant protein kinase is selected. The protein kinase three-dimentional structure employed can be that of the PDK1 mutant protein alone or in the presence of a compound binding to the allosteric HM/PIF-binding pocket and/or with a compound binding to the ATP binding site, or both. The three dimentional structures can be those obtained following the crystallization of the mutant PDK1 protein. Compounds, both allosteric compounds or those binding to the ATP binding site can be either soaked into crystals of the mutant protein or preincubated with the mutant protein previous to setting the crystallization condition.

Three-dimentional structures that are compared may be predicted or modeled three-dimentional structures. For example when compounds are compared with the structure of the mutant protein kinase of the invention, the structures of multiple possible three-dimentional structures of a compound may be compared with the chances of interacting with a given site, for example the ATP binding site or the HM/PIF-binding pocket site on the mutant protein kinase.

The mutant PDK1 protein kinase of the invention may be used in tests in the presence of protein substrates of PDK1. The substrates of PDK1 may be short polypeptides comprising the direct region interacting with the substrate binding site or longer polypeptides, comprising the substrate binding site and a docking site to interact with the allosteric HM/PIF-binding pocket. Alternatively, the substrate protein may comprise a substantially complete domain, or a full length protein. For the purpose of co-crystallization with PDK1, it may be useful that the substrate is mutated in a way that would interact with higher affinity to PDK1. A kinase dead mutant of the substrate or a mutant substrate which has the PDK1 phosphorylation site mutated to a non-phosphorylatable residue may be of advantage for the co-crystallization procedure. Other mutations in the substrate may involve the mutation at the HM phosphorylation site, for example to Asp or Glu or at the Turn-motif/Z-phosphorylation site, for example to Asp, Glu or Ala.

It can be reasonably expected that the use of the mutant PDK1 protein of the invention in combination with a substrate polypeptide may allow the co-crystallization of the complex and further allow to identify or better define sites which may be used for drug design.

Co-crystallization and structures determined from co-crystallized molecules are useful in molecular modeling and in determining features of the complex interaction that are important. This can be useful in designing or selecting compounds.

It is preferred that the compounds bind to the HM/PIF-binding pocket and / or to the ATP binding site. Compounds that bind to the ATP binding site may also protrude and prompt interactions outside the ATP binding site.

Compounds that bind to the HM/PIF-binding pocket can be small molecular weight compounds and / or polypeptides and / or peptidemimetics.

It can also be envisaged that the protein kinase mutant of the invention can be put in contact with a substrate or pseudo-substrate polypeptide in the presence or absence of an ATP competitor and / or an allosteric compound. This may be of interest to display a protein in a conformation most resembling the active structure which requires the simultaneous interaction with substrates (which must take place with the simultaneous binding of molecules at the ATP binding site and to the substrate binding site).

The present invention thus provides
(1) 1. A mutant protein kinase derived from a starting protein kinase having a hydrophobic pocket in the position equivalent to the hydrophobic PIF-binding pocket defined by the residues Lys115, Ile118, Ile119, Val124, Val124, Val127 and/or Leu155 of full length human PDK1 shown in SEQ ID NO:1 and having a phosphate binding pocket equivalent to the phosphate binding pocket defined by the residues Lys76, Arg131, Thr148 and/or Gln150 of full length human PDK1 shown in SEQ ID NO:1, wherein said mutant protein kinase has a at least two mutations in one of its motives equivalent to AGNEYLIFQK (SEQ ID NO:2) and
   LDHPFFVK (SEQ ID NO:3) of human PDK1, or a fragment or derivate thereof.
(2) a polynucleotide sequence encoding the mutated protein kinase of (1) above;
(3) a vector comprising the polynucleotide sequence of (2) above;
(4) a host cell transformed with the vector of (3) above and/or comprising the polynucleotide sequence of (2) above;
(5) a process for producing the mutated protein kinase of (1) above which comprises culturing the host cell of (4) above and isolating said mutated protein kinase;
(6) a method for identifying a compound that binds to the PIF-binding pocket allosteric site of a starting protein kinase as defined in (1) above, which comprises the step of determining the effect of the compound on the mutated protein kinase of (1) above or the ability of the compound to bind to said mutated protein kinase;
(7) a kit for performing the method of (6) above which comprises a mutated proten kinase of (1) above; and
(8) a compound identified by the method of (6) above binding to the PIF-binding pocket allosteric site of a starting protein kinase.

### Short description of the Figures

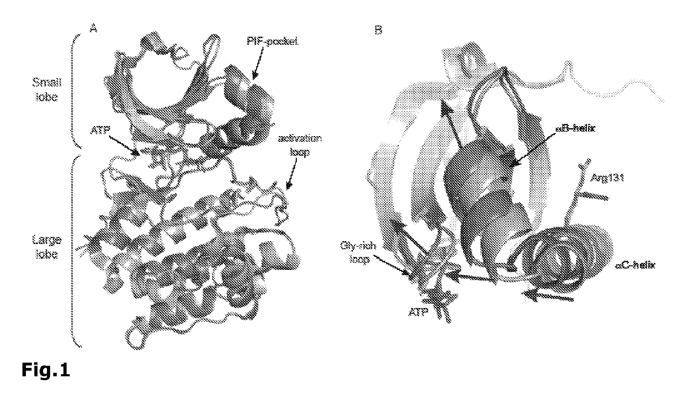

Figure 1: Superimposition of the original structure and the crystal form II of PDK1. Superimposition of 1H1W (grey) with crystal form II (blue) was performed on the backbone atoms of the C-terminal lobe. (A) The large domain secondary structures perfectly surperimpose in both structures as well as the ATP molecule. The tip of the activation loop is also disordered although the segments that could be modeled show a conformational change. The secondary structures in the small lobe appear very similar; however there is a shift. The movement is described in (B) that represents a close-up on the secondary structures of the PIF-pocket. The dark blue arrows indicate the direction is which the secondary structures in the small lobe are displaced. Most importantly, the α-B- and α-C-helices seem to be rotated clockwise compared to the original structure. The Gly-rich loop is also displaced in the novel crystal form.

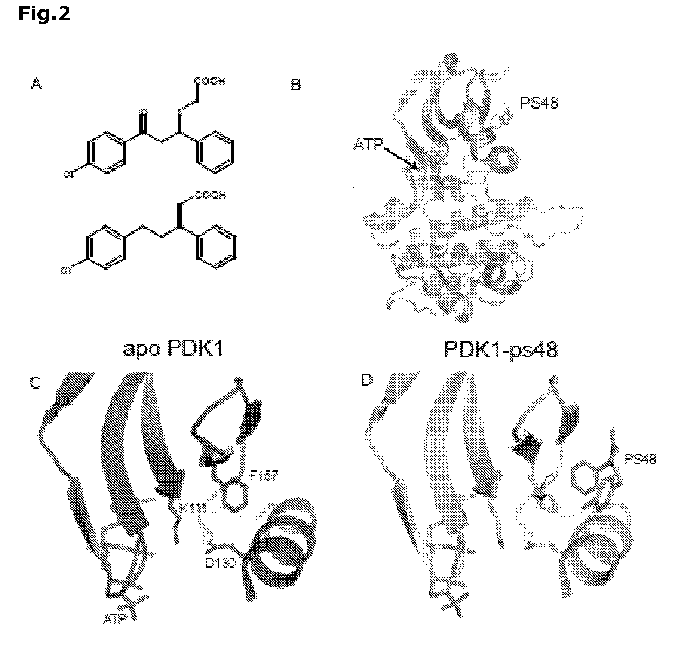

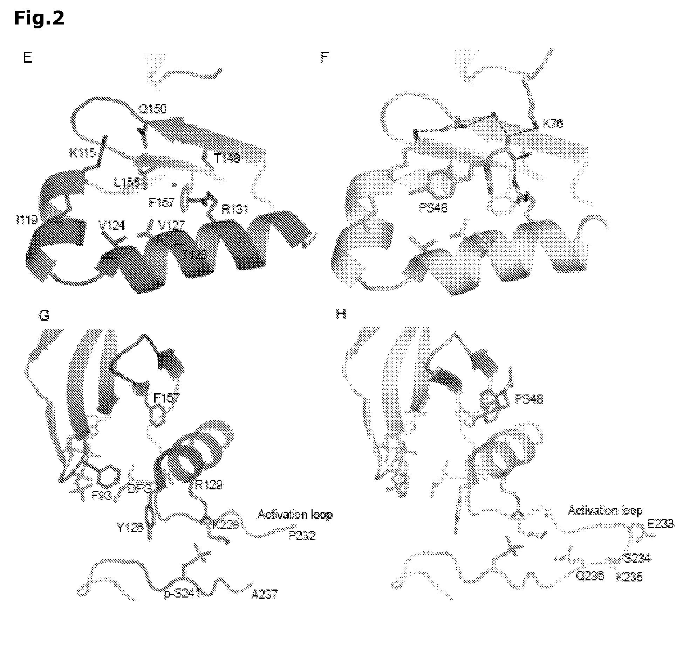

Figure 2: Crystal structure of apoPDK1 and PS48 bound PDK1 in the crystal form II. (A) Compounds PS48, and compound 1 (Engel et al. 2006) formulas. (B) Superimposition of PDK1-ATP and PDK1-ATP-PS48 structures on their C-terminal lobe. (C,D) Binding of PS48 to the PIF-pocket triggers a conformational change in residues Phe157 and the ion pair Lys111-Glu130. (E,F) The PIF-pocket and phosphate-binding pocket in PDK1-ATP and PDK1-ATP-PS48 structures. The phenyl rings in PS48 form interactions to the hydrophobic residues lining the PIF-pocket while the carboxylate group coordinates the residues forming the phosphate-binding pocket. Gln150, Lys76, Arg131, Thr128 have changed conformation. (G,H) The a-C-helix links together the PIF-pocket, the ATP binding site and the activation loop. In the PDK1-ATP-PS48 structure the activation loop is ordered. New interactions, between Lys228 and Gln236, and between Lys228 and Arg129, mediated by water molecules, are formed in the complex.

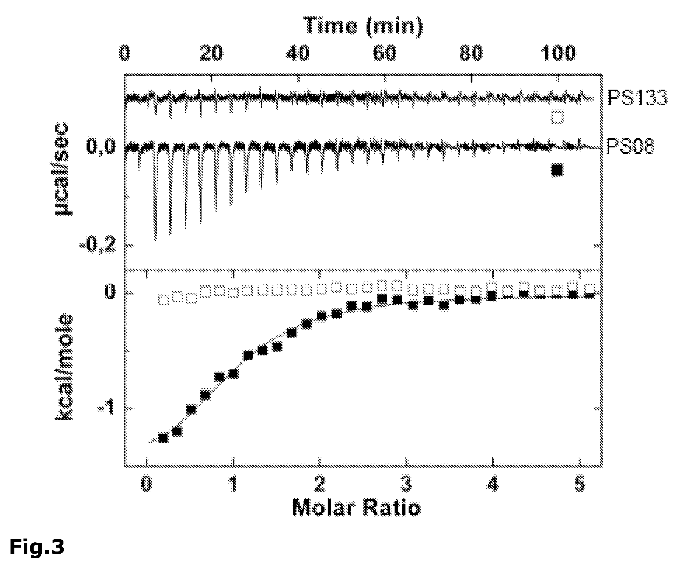

Figure 3: Characterization of PS08 and PS133 isomers interactions with PDK1₅₀₋₃₅₉ by ITC. The top panel shows the raw heat signal for successive injections of compound PS08 solution and compound PS133 solution into a PDK1₅₀₋₃₅₉ solution at 20°C. The bottom panel shows the integrated heats of injections corrected for heats of dilution for compounds PS08 (filled squares) and PS133 (open squares), with the solid lines corresponding to the best fit of the data using Origin^{™} software. No binding of compound PS133 to PDK1₅₀₋₃₅₉ is detected in conditions where compound PS08 shows clear binding. Thermodynamic parameter values are given in Table III.

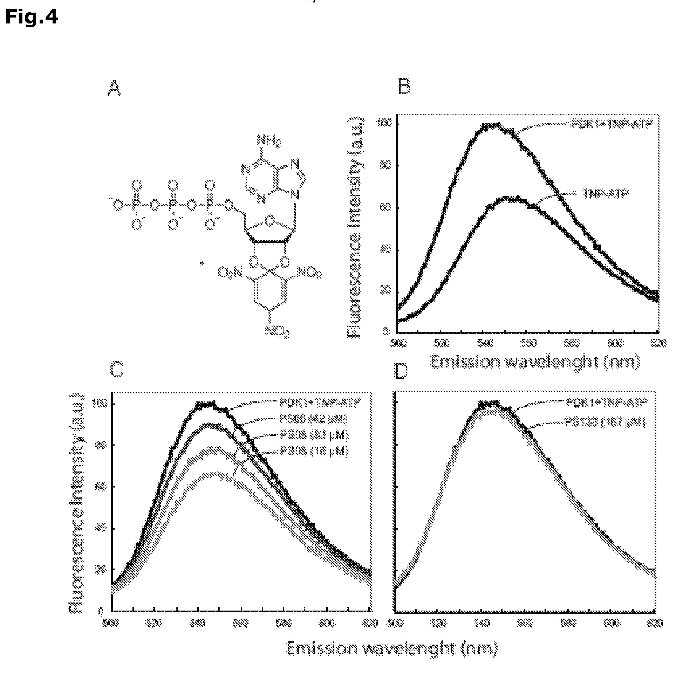

Figure 4: Modulation of PDK1 conformation by small compounds. Effect PS08 and PS133 on the emission fluorescence of trinitrophenyl-ATP (TNP-ATP) / PDK1. Fluorescence intensities are expressed as % of the maximal fluorescence intensity of TNP-ATP obtained with addition of 15 µM PDK1 CD. Addition of PS08 to the TNP-ATP-PDK1 mixture triggered a concentration dependent decrease of the fluorescence intensity. At 167 µM PS08, the maximum fluorescence intensity was 60 %, similar to TNP-ATP alone, suggesting that the interaction with PS08 may have induced a change in PDK1 conformation that was not compatible with TNP-ATP binding. In contrast, addition of 167 µM PS133 to the TNP-ATP-PDK1 mixture did not produce any decrease in TNP-ATP fluorescence, thereby suggesting that PS133 was not able to produce a similar conformational change in PDK1.

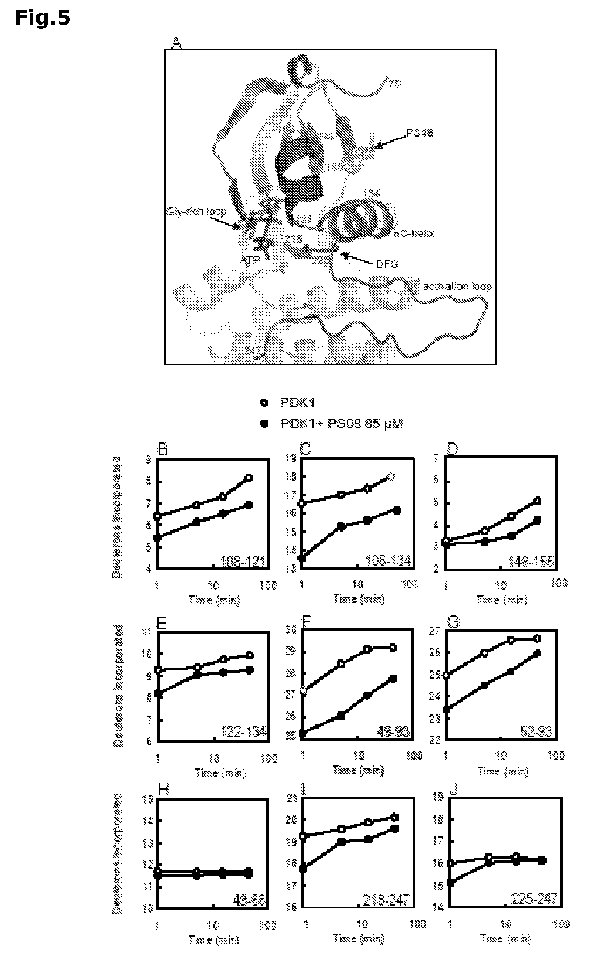

Figure 5: Deuterium incorporation levels along time in presence or absence of PS08 for regions in PDK1 that have shown protection. (A) Catalytic domain of PDK1 bound to PS48. (B,C,D,E,F,G,H,I,J) Deuterium incorporation levels at 1, 5, 15 and 45 s in protected peptides in PDK1 (open circles) and in PDK1-PS08 samples (closed circles). (B) Peptide 108-134, that comprises two thirds of the β3-strand, the α-B-helix (red) and the α-C-helix (orange), incorporated 4 ²H less in the presence of PS08 at the first time point and 2 ²H at following time points. (C) Peptide 108-121 (red) had decreased incorporation of 1 ²H in the presence of PS08. (D) Peptide 122-134 (orange) corresponding to the α-C-helix incorporated 1 ²H less in the presence of PS08 at the first time point. Overlapping peptides 49-93 (E) and 52-93 (F) comprising residues from the N-terminus up to Phe93 in the Gly-rich loop (pink) were strongly protected (1.5 to 2 ²H protection in the presence of PS08) by low molecular weight compounds but not the shorter peptide 49-66 (G) suggesting that the conformational change occurs in the 67-93 segment. This results correlates with the conformational change of Phe93 observed in the structure of PDK1 bound to ps48 in comparison to apoPDK1. (H) Peptide 146-155 (yellow) encompasses β-4-and a third of β-5-strand incorporated 1 ²H less in the presence of small molecular weight compounds was observed at time points 15 and 45 s. (I) Peptide 218-247 (blue), comprising the entire activation loop from the magnesium binding loop until the P+3 site, incorporated 1.5 ²H less at the first time point in the presence of PS08 compared to PDK1 alone and 0.5 to 1 ²H less in the following time points. Overlapping peptide 225-247 (J) starting next to the DFG motif (dark blue) is similarly protected at first time point but then does not further exchange ¹H for ²H suggesting that the conformational change takes place in the 218-225 segment.

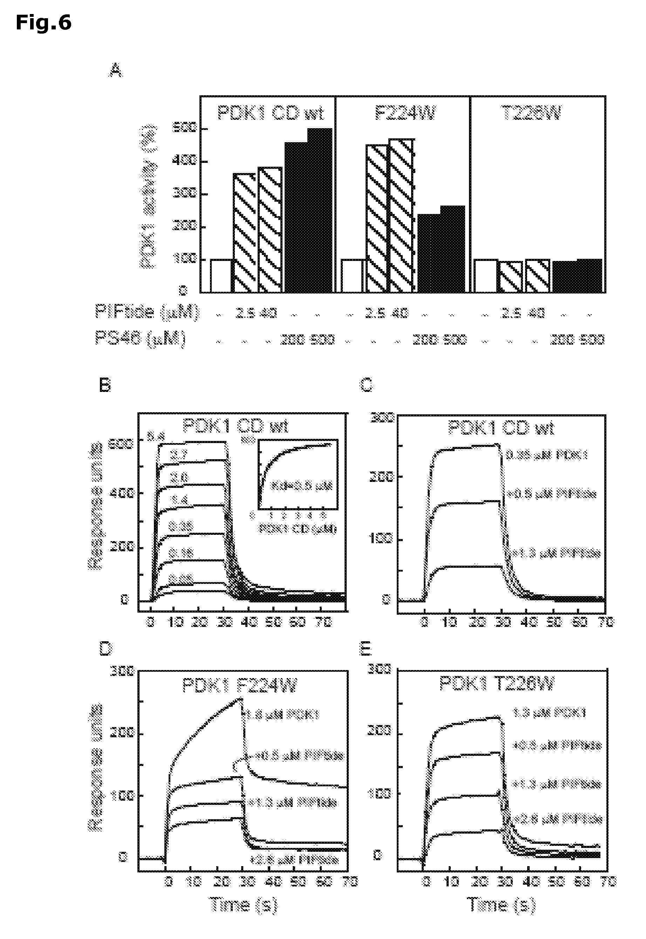

Figure 6: Mutation at Thr226 in PDK1 uncouples the ligand binding to the HM/PIF-pocket from activation of the kinase. The catalytic domain (CD) of PDK1 50-359 and its mutants Phe224Trp and Thr226Trp were expressed in insect cells and purified. (A) PDK1 activity was measured in the presence or absence of the HM-polypeptide PIFtide or PS46 using T308tide as a substrate. PDK1 CD wt and PDK1 [Phe224Trp] were activated by PIFtide and PS46 whereas PDK1 CD [Thr226Trp] mutant was not activated. (B,C,D,E) PDK1 [Phe224Trp] and PDK1 [Thr226Trp] retain significant binding to P-HM polypeptides and PIFtide. Surface plasmon resonance measurements were carried out on a BiaCore system to evaluate the binding affinities of PDK1 CD wt, PDK1 [Phe224Trp] and PDK1 [Thr226Trp] to HM-polypeptides. Biotin-P-HM-polypeptide derived from S6K1 was immobilized on a streptavidin-coated sensor chip and the specific interaction with PDK1 was recorded. (B) The direct binding of PDK1 CD wt to biotin-P-HM-S6K was recorded at the indicated concentrations of PDK1 CD. The Kd (0.5 µM) was estimated based on the response units obtained at each concentration at equilibrium. (C,D,E) PDK1 CD (50-359), wt (0.35 µM), PDK1 [Phe224Trp] or PDK1 [Thr226Trp] were injected onto the system alone or in the presence of the indicated concentrations of PIFtide.

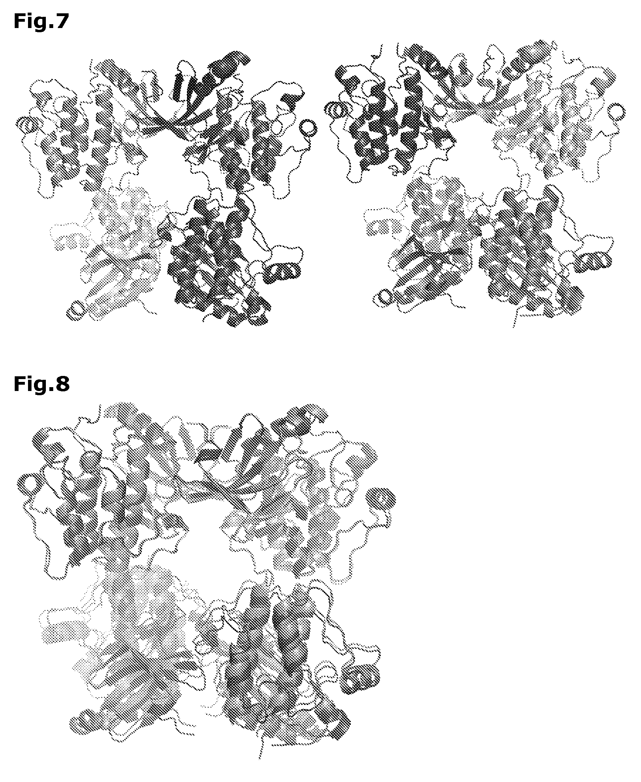

Figure 7: Crystal packing in the forms III and IV. The two molecules forming the crystallographic dimer in PDK1_30 (left) are represented in orange and their symmetric molecules in red were generated in Pymol. In PDK1_33 (right), the four molecules in the ASU are represented in a shade of purple. Although the crystal forms were distinct, the crystal contacts appeared very similar as all four molecules superimposed to the other structure.
Figure 8: Superimposition of the crystal forms III and IV highlights a shift of the molecules forming the crystallographic tetramer between the two structures. One crystallographic dimer of PDK1_33 (purple) was superimposed to the crystallographic dimer in PDK1_30 (orange). The symmetric dimer in PDK1_30 (orange) was generated in Pymol. The second crystallographic dimer of PDK1_33 superimposed the generated dimer of PDK1_30. However, there was a shift in the latter two dimers that put the four molecules slightly closer together in the PDK1_30 structure. This could explain that they belonged to two different crystal forms. A careful study of individual intermolecular crystal contacts showed no difference, thus we assumed that the movement was due to the presence of polypeptides of different lengths between the molecules.

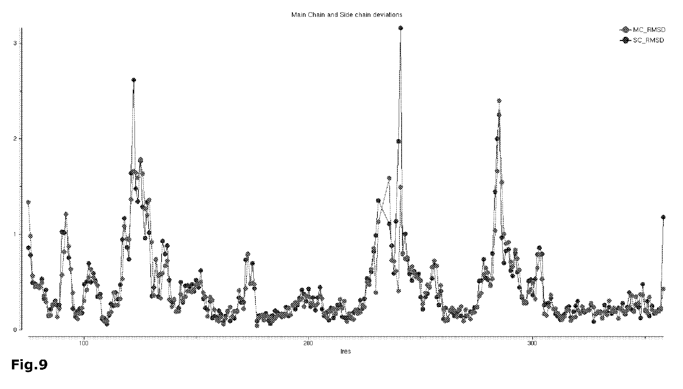

Figure 9: RMS deviations between the crystal forms III/IV and the crystal form II. (A) Main-chain and side-chain RMS deviations between PDK1_33 and PDK1_23. The RMSD were calculated on main-chain atoms (red) and side-chain atoms (blue) for all residues in PDK1. The large deviations around residues 240-250 correspond to disordered residues in the activation loop. The largest deviations are observed in the small lobe and most importantly in the Gly-rich loop, the β2- and β3-strands, and the αB- and αC-helices. A large RMS deviation is also observed in the αG-helix and in the adjacent loop. This is likely due to the fact that the α-helix is involved in a distinct crystal contact in the crystal forms III and IV as in the crystal form II although it could also be a consequence of the activation loop conformational change.

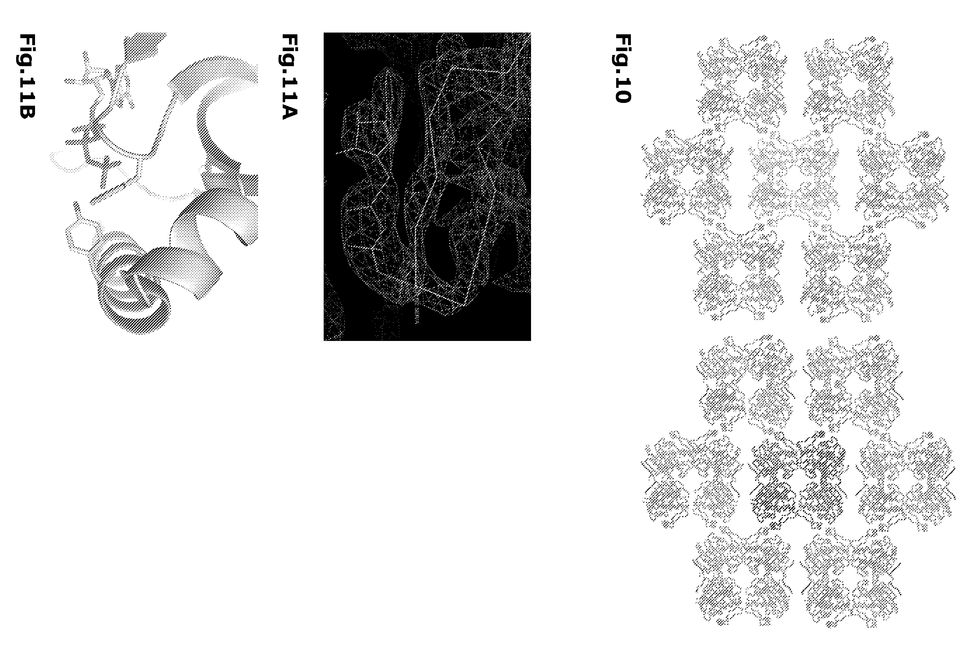

Figure 10: Comparison of packing III and IV. In the PDK1_33 structure (right), the peptide derived from the PKB-HM could be partially modeled (red). This peptide is localized in the void separating molecules in the crystals. In the PDK1_30 structure (left), PIFtide could not be modeled. PIFtide is nine amino acids longer that HM-PKB. This may be why the space between different tetramers is wider in PDK1_30.

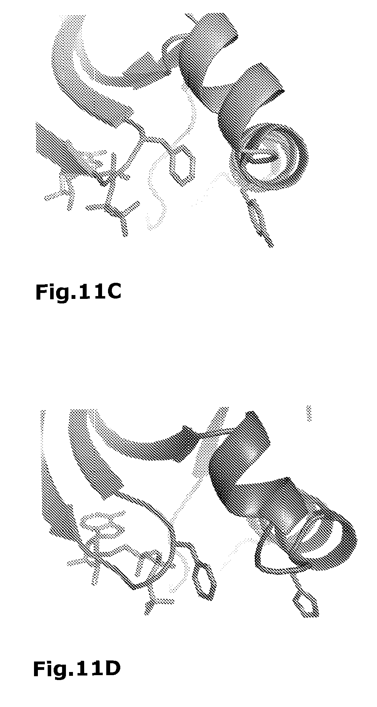

Figure 11: The ATP conformation in PDK1_30 is different to previously described PDK1 structures and resembles most the ATP conformation in the "closed" structure of PKA. (A) The ATP and Gly-rich loop are represented with the |2F_{obs}-F_{calc}| electron density map in this region. The density map, at 2 σ level, showed very clearly that the ATP conformation was distinct from previous PDK1 structures. (B) In PDK1_30, the conformation of the ATP molecule is similar to the ATP conformation in the "closed" PKA structure (D) as the base and the α- and β-phosphates superimpose the equivalent ones in PKA. The γ-phosphate points in the opposite direction than its equivalent in PKA. (C) The conformation of the phosphates of the ATP molecule in PDK1_8, which is similar to all other PDK1 structures solved previously, is different to the one of PDK1_30. Interestingly, the Tyr126 appears in a completely different conformation than in other PDK1 structures as it points towards the phosphor atom of the β-phosphate.

### Sequence Listing - Free Text

- SEQ ID NO:: Description
- 1: full length human PDK1
- 2-3: Motives of human PDK1
- 4: mutated hPDK1₅₀₋₃₅₉
- 5: mutated hPDK1₆₇₋₃₅₉
- 6: mutated hPDK1₅₀₋₃₅₉ with His-tag and TEV cleavage site
- 7: hPDK1₅₁₋₃₅₉ with two additional N-terminal aa residues
- 8-10: substrates for protein kinase PDK1
- 11-12: Candida albicans PDK1 sequences Ca93274 and Ca97297

### Detailed description of the Invention

Aspect (1) of the invention pertains to a mutant protein kinase derived from a starting protein kinase having at least two mutations in one of its motives AGNEYLIFQK (SEQ ID NO:2) and LDHPFFVK (SEQ ID NO:3).

The "starting protein kinase" according to the inventions can be derived from a mammalian protein kinase grouped within the AGC group of protein kinases, such as SGK, PKB, S6K, MSK, RSK, LAT, NDR, MAST, ROCK, DMPK, MRCK, PKA, PKG, GRK, PRK, PKC and their isoforms, or Aurora or YANK protein kinases and their isoforms, among which those derived from a PDK1 protein kinases are preferred, or it can be derived from infectious organisms such as Candida spieces such as Candida albicans PDK1 (including those shown in SEQ ID NOs11 and 12), *Aspergillus* spp., *Cryptococcus neoformans, Histoplasma capsulatum,* and

*Coccidioides.* Most preferably the "starting protein kinase" is derived from the human protein kinase PDK1 having SEQ ID NO:1.

In a preferred embodiment of aspect (1) of the invention the mutant protein kinase has a mutation in the motif of SEQ ID NO:2, notably the mutation is a non-conservative mutation; and/or is a mutation of the residues Y or Q. A "non-conservative mutation" within the invention includes a replacement of a hydrophobic amino acid residue with a polar or ionic amino acid residue and *vice versa,* and/or of a large amino acid residue with a small one and *vice versa.* For the motif of SEQ ID NO:2 the mutation of the residue Y with G or a mutation of the residue Q to A is preferred, most preferably said motif has the Y to G and Q to A mutations.

In a further preferred embodiment of aspect (1) of the invention the mutant protein kinase has a mutation in the motif of SEQ ID NO:3, notably the mutation is a non-conservative mutation; and/or is a mutation of the residues D, H, P, or K.

For the motif of SEQ ID NO:3 the mutation of the residue D or K with M, H or P is particularly preferred.

In a particularly preferred embodiment of aspect (1) of the invention said starting protein kinase is human PDK1 shown in SEQ ID NO:1 and said mutant protein kinase has at least two mutations at a position corresponding to positions Tyr288 Gln292, wherein the numbering refers to the full length human PDK1 shown in SEQ ID NO:1, or a fragment or derivate thereof. The mutant protein kinase may have one or more further point mutations at positions corresponding to Lys296, Asn296 and Ile295.

In a further particularly preferred embodiment of aspect (1) the mutant protein kinase has the mutations Tyr288 Gly and Gln292Ala, wherein the numbering refers to the full length human PDK1 shown in SEQ ID NO:1, preferably the mutant protein kinase has the the sequence shown in SEQ ID NOs: 4, 5 or 6.

The "fragment" of the mutant protein kinase may be any C- and/or N-terminally truncated mutant protein kinase provided that it comprises the the hydrophobic PIF-binding pocket, the phosphate binding pocket and the mutated motives of SEQ ID NOs: 2 and 3 and has a protein kinase activity comparable with that of the full length protein kinase.

The "derivative" of the mutated protein kinase includes C- and/or N-terminal fusion products with a peptide or protein sequence (such as leader and expression sequences, sequences suitable for purification and processing of the mutant protein kinase and other functional protein sequences) and with low molecular chemical compound (such as PEG, marker molecules, protective groups).

In a particular preferred embodiment of aspect (1) of the invention the mutated protein kinase is in a crystalline form.

Protein phosphorylation provides organisms with a fine tuning mechanism to regulate cell fate, for example, in response to external stimuli. Importantly, it is widely recognized that loss of regulation of protein kinases can lead to disease states, including cancer, neurological disorders and diabetes. In order to cure and treat diseases where protein phosphorylations are misregulated, a considerable effort is currently directed to the development of protein kinase inhibitors. In contrast, the phosphorylation-dependent conformational changes on key proteins and enzymes are not normally recognized as targets for drug development. This is in part due to the fact that the molecular mechanisms by which phosphorylation prompts conformational changes in proteins are widely unknown. Throughout the years, we and others characterized the HM/PIF-bionding pocket in AGC kinases as the key site target of the phosphorylation-dependent conformational change. More recently, we focused on the PIF-binding pocket on PDK1 and developed low molecular weight compounds which can allosterically activate PDK1 *in vitro.* In the present work, we provide crystallographic data describing the binding of low molecular weight compounds to the PIF-binding pocket in PDK1 and further provide data on the conformational change which activates the kinase. Thus, our crystallography work showed that the small compounds indeed bound to the target site prompting subtle conformational changes within the pocket, the neighbour phosphate binding site, the glycine-rich loop and the activation loop. Furthermore, using a fluorescent ATP analogue as a conformational probe for the ATP binding site, our data provided evidence for the conformational change at the ATP binding site upon the occupancy of the allosteric PIF-binding pocket by compounds. Finally, we confirmed those results employing ¹H/²H exchange followed by mass spectrometry. Altogether our results supported a model where small compounds bound to the PIF-binding pocket regulatory site and prompted modifications locally and allosterically on the ATP binding site, where residue 226, directly neighbouring the highly conserved DFG motif at the start of the activation loop, participated in transducing the binding to the PIF-binding pocket into the conformational change which activated the enzyme. We believe that this is the first report on the molecular characterization of the binding and conformational transitions induced by low molecular weight compounds designed to mimic phosphorylation-dependent conformational changes.

We first approached the molecular understanding of the mechanism of activation by low molecular weight compounds by crystallography. In our assays, the standard PDK1 construct crystallized only in one crystal packing which impeded the co-crystallization or soaking with small compounds. We therefore explored diverse alternative strategies and found that the inclusion of two mutations (Tyr288Gly and

Gln292Ala) which participated in a crystallographic contact occupying the PIF-binding pocket from a neighbouring molecule, rendered a PDK1 50-359 mutant protein which could be concentrated to higher levels (28mg/ml) and crystallized in a different crystal packing (crystal packing II). Surprisingly, the high resolution structure revealed that the PDK1-ATP complex was strikingly similar to the structure of PDK1-ATP solved on crystal packing I. It is possible that ATP, in the absence of Mg2+ stabilized this PDK1 conformation, as staurosporine stabilized a very similar "intermediate" conformation in PKA. Alternatively, it can be envisaged that the catalytic domain of PDK1 is particularly stable in this conformation. The small compound bound to the PIF-binding pocket mainly by hydrophobic interactions between the phenyl rings and the hydrophobic pocket and by the specific interaction of the carboxylate with residues forming part of the phosphate binding site neighbouring the PIF-binding pocket.

Most of the interaction energy was provided by the entropic component (80%). The favorable entropy term results from the balance between the dehydration of hydrophobic and charged or polar groups both from PS48 and the PIF-pocket (AS > 0) and the conformational entropy (AS < 0). The crystal data shows that at least one water molecule is expelled from the PIF-pocket and one is displaced to coordinate interactions between PS48 carboxylate and polar side chains in the phosphate binding site upon ps48 binding (Fig. 2E and 2F). On the other hand, the conformational entropy implied the stabilization of a different conformer for Phe157 and the stabilization of the side chains of Arg131 and Lys76. This binding further prompted allosteric conformational changes on the ATP binding site, as revealed in the crystal complex with PS48, in the TNP-ATP fluorescence data and in the deuterium exchange protection. In parallel, the compound binding also prompted some degree of increased entropy within the α-C-helix, which had increased B-factors in the presence of PS48. Altogether, the entropy involved in the burial of the hydrophobic phenyl rings and dehydration of the PIF-pocket and the compound was the driving force of the interaction, in spite of the conformational entropy involved.

The PDK1-ATP-PS48 crystal structure provided molecular data on the specific conformational changes observed on the crystal upon binding to PS48. The extent of the allosteric effect on the ATP binding site was further manifested on the fluorescent test developed to evaluate the ATP binding site conformation. In this assay, an increase in fluorescence intensity reflected the binding of the TNP-ATP to PDK1, and the addition of HM polypeptide or compounds activating the kinase prompted a diminution in the fluorescence intensity. Therefore, one possibility is that TNP-ATP bound to the inactive structure of PDK1 and that this binding was lost in the active/compound-bound conformation of the kinase; alternatively, binding of the compound to the PIF-binding pocket could increase the OFF rate of TNP-ATP, switching equilibrium towards unbound TNP-ATP, decreasing fluorescence intensity.

The amide ¹H/²H exchange and mass spectrometry assay on PDK1 showed a strong protection of the PIF-binding pocket by PIFtide and also by the activating low molecular weight compounds, further corroborating that, also in solution, the PIF-binding pocket is the binding site for both activators. Notably, even if both PIFtide and the low molecular weight compounds prompted a significant and constant level of protection on the α-B-helix, the protection on the α-C-helix was important (5 ¹H) but also very transient in the presence of PIFtide (see Supplementary Fig.3). Most surprisingly, the polypeptide which comprised exclusively the α-C-helix (122-134) had almost 10 ¹H exchanged after the first time point (over a maximum of 13 exchangeable ¹H in the amino acid sequence), implicating the existence of a region of relative high exposure to the solvent, inconsistent with a stable α-helix. Based on this, it is tempting to speculate that the crystal structures of PDK1 so far obtained are in an "overall active" conformation, and that the inactive protein in solution would have considerably higher disorder of the α-C-helix.

The amide ¹H/²H exchange experiments also identified the polypeptide 67-93, including the Gly-rich loop sequence as a region protected from ¹H/²H exchange by activators. This is consistent with the crystal structure data that showed Phe93 to be positioned differently in the apo and in the compound-bound structures. This region encompasses the "top" of the ATP binding site and is a clear allosteric effect product of the activator binding to the PIF-binding pocket. Also in the PDK1 crystal structure complexed to PS48, we identified allosteric changes on a key ATP binding site residue, Lys111 (amino group Nζ away from its position in the apo structure), and on the region corresponding to amino acids 218-224 ending at the Phe224 from the DFG motif. The existence of allosteric conformational changes on the ATP binding site were supported by the use of the ATP conformational sensor, TNP-ATP, which identified a gross decrease in fluorescence upon the addition of low molecular weight compounds. The effect was specific since PIFtide and phosphorylated HM polypeptides also prompted a similar decrease. Furthermore, the specificity was highlighted by the fact that the mirror image compound did not prompt any significant decrease in fluorescence intensity. Thus, the results established the existence of an allosteric conformational change at the ATP binding site upon the binding of compounds to the PIF-binding pocket and further identified molecular effectors of the conformational change.

Finally, the amide ¹H/²H exchange experiments identified an allosteric protection on the α-G-helix on the bottom of the large lobe, some 30 A away from the activator binding site. The allosteric effect may be transduced by the activation loop (that was ordered in the PDK1-compound crystals) via its continuation, the P+1 loop. In another AGC kinase, PKA, the α-G-helix participates in the binding of the regulatory subunit (Kim, C. et al., Cell 130:1032-1043 (2007)). We have also identified turn-motif/zipper phosphorylation of full length PKCzeta allosterically protected the α-G-helix from ¹H/²H exchange. Since this helix is used for intra- and inter- molecular interactions, it is possible that the present finding on PDK1 does not represent an allosteric effect related to the activation mechanism but related to subtle conformational changes that may take place during the process of interaction and release from physiological substrates. However, we cannot discard that this protection may be an artefact or unspecific binding of small compounds since we have observed this effect only with one methodology.

Finally, we evaluated the effect of mutating residues within the DFG motif (Phe224Trp) and the start of the activation loop (Thr226Trp). We predicted that the mutation of PDK1[Thr226] to Trp may not have major detrimental effects since the equivalent residue in other AGC kinases is Phe, Leu or Met, while it is Trp in Aurora protein kinase, which is most closely related to AGC kinases and may share an analogous mode of regulation by interaction with TPX2 (Bayliss, R. et al., Mol. Cell 12:851-862 (2003)). In agreement with this, Thr226 has a reasonably high basal activity. Nevertheless, in spite of binding PIFtide with high affinity, PDK1[Thr226Trp] was not activated by PIFtide. Thus, we believe that this residue can uncouple the binding to the PIF-binding pocket from the activation of the enzyme. We can speculate that the molecular impediments in the activation process of PDK1[Thr226Trp] are related to the contiguous DFG motif, which could mediate allosteric conformational changes to the ATP binding site. An importance of the DFG motif in the activation mechanism is in agreement with the key role ascribed to this motif in the activation process of PKB (Yang, J. et al., Nat. Struct. Biol. 9:940-944 (2002)).

It is likely that the current model by which the phosphorylation transduces a conformational change via an inter or intra-molecular interaction depending on phosphorylation may be wide-spread in nature. Our current work suggests that such regulatory mechanisms may be targeted by small compounds, with potential to become orally available drugs. Whereas, general protein-protein interactions in the cell may be very stable to be competed pharmacologically by small compounds, phosphorylation-dependent interactions may be more amenable for drug development. Based on the present results, we envisage that, through understanding the molecular mechanism by which a protein is regulated via phosphorylation, it may be possible to rationally design small molecular weight drugs to mimic conformational states physiologically achieved by phosphorylation. Furthermore, the understanding of the molecular mechanisms involved in the allosteric regulation of proteins by other post-translational modifications may also provide new avenues for future drug development.

The invention is specifically described in the following non-limiting examples.

### Examples

### Materials and Methods

Complete protease inhibitor cocktail tablets were from Roche. Protein concentration was estimated using a coomassie reagent (Coomassie Plus) from Perbio. Protein was concentrated using Vivaspin concentrators. Glutathione adn Ni-NTA chromatography resins were from GE Healthcare or Chromatrin Ltd. Human embryonic kidney 293 cells (ATCC collection) were cultured on 10 cm dishes in Dulbecco's modified Eagle's medium containing 10% fetal bovine serum (Gibco). Mammalian tissue culture materials were from Greiner. Insect cell expression system and all insect cell related material were from Invitrogen and were used as recommended by the manufacturer. Molecular biology techniques were performed using standard protocols. Site-directed mutagenesis was performed using a QuikChange stratagy (Stratagene) following the instructions provided by the manufacturer. DNA constructs used for transient transfection were purified from bacteria using a Qiagen plasmid Mega kit according to the manufacturer's protocol.

DNA sequences were verified by automatic DNA sequencing (Applied Biosystems 3100 Genetic Analyzer). The polypeptide substrate of protein kinase PDK1 was T308tide (KTFCGTPEYLAPEVRR (SEQ ID NO:8); > 75% purity) which is derived from the PDK1 phosphorylation site on PKB. The polypeptides used to bind PDK1 were PIFtide (REPRILSEEEQEMFRDFDYIADWC; SEQ ID NO:9), which is derived from the C-terminal 24 amino acids of the PDK1 substrate PRK2 (synthesized by JPT Peptide Technologies GmbH) and a phosphorylated HM polypeptide derived from the PDK1 substrate S6K KQTPVDS(P)PDDSTLSESANQVFLGFT(P)YVAPSV; SEQ ID NO:10) (Hauge, C. et al., Embo. J. 26:2251-2261 (2007)). The biotin-(P)HM S6K peptide was a gift from Morten Frödin (Copenhagen, Denmark).

Expression and purification of protein kinases: PDK1 used for crystallographic studies was prepared essentially as previously described (Engel, M., Embo. J. 25:5469-5480 (2006)). The double mutant PDK1 protein was more soluble than the wild type counterpart and could be concentrated up to 25mg/ml.

Crystallization of PDK1 50-359: Wild type PDK1₅₀₋₃₅₉ (SEQ ID NO:7) and mutant (Tyr288Gly-Gln292Ala) (SEQ ID NO:4) were produced and concentrated to 8.5 mg/ml and 28 mg/ml respectively. High throughput screenings for crystallization conditions were performed at 18°C using a Cartesian Technology workstation which allows sitting nanodrops (200 nl of protein + 200 nl of mother liquor) in Greiner 96-well plates (CrystalQuick™ Sitting Drop Protein Crystallization Plates, Sigma-Aldrich). Crystals were obtained and reproduced in 24-well plates in the presence of ATP (5 mM) using the hanging drop method by mixing 1.5 µL of protein solution and 1.5 µL of the reservoir solution containing for the wild-type protein 2.2 M Ammonium Sulfate, 0.1 M Tris-HCl pH 8.5 and 10% glycerol and for the mutant 1.4 M Sodium Citrate, 0.1 M Sodium HEPES pH 7.5. Soaking experiments were performed on both crystal forms with various compounds at different concentration (2-5 mM). The crystals of the wild type PDK1 were similar to previously described (Biondi, R. M. et al., Embo. J. 21:4219-4228 (2002)). The crystals in the crystal packing II grew to a maximum dimension of 0.03 mm x 0.05 mm x 0.07 mm within 3 days at 18°C. Crystals are in the monoclinic space group C2. The crystals were frozen in liquid nitrogen.

Data collection, structure solution and refinement: Data up to 2.5 Å resolution were collected using either a Rigaku Micromax 007 rotating anode generator, equipped with a MAR-345 image plate detector system (MAR USA) and a nitrogen cryostream (Cryojet Oxford Instruments, Oxford Cryosystem) or 1.9 Å using a synchrotron source at ESRF (European Synchrotron Radiation Facility, Grenoble) on beamlines ID14-EH2, ID14-EH3 and ID23-EH1. Data integration and reduction were carried out with the programs XDS (Kabsch, 1993) and SCALA in the CCP4 package (Kabsch, 1988; Evans, 1993). Molecular replacement performed with Phaser (Read, 2001) placed one molecule in the asymmetric unit. Structure refinement was done in Refmac (Murshudov et al, 1997) alternated with rounds of validation and rebuilding with the program Coot.

Water molecules were introduced in the model with the program ArpWarp (Perrakis et al, 1999). The refined coordinates for the final model (Table I) have been deposited in the PDB with accession code XXXX.

Deuterium Exchange: The PDK1 50-359 [Y288G;Q292A] samples were incubated in deuterated water ²H₂O at 30°C and the mass of the protein evaluated at the stated incubation times and the exchange stopped by quenching with acid and incubation on ice. For local exchange analysis, the labeled proteins were digested with pepsin and the resulting polypeptides analyzed by tandem mass-spectrometry (LC-MS/MS) for their incorporation of deuterium atoms. The graphics represent the number of deuterium atoms incorporated into each polypeptide upon the stated incubation time in deuterated water. The PDK1 50-359 [Y288G;Q292A] samples were incubated in deuterated water (²H₂O) at 30°C. Labeling reaction started after mixing of labeling buffer (25 µM Tris, NaCl 150 µM, MgCl 5mM, DTT 1mM, glutathione 2.5 mM, pH 7.1) and the protein sample. Over time, amide hydrogen atoms exchanged for deuteriums and the molecular weight of the protein increased. We repeated the ¹H/²H exchange experiments at four different incubation times: 1, 5, 15 and 45 minutes. At the four incubation times, aliquots of the exchange reaction were quenched by addition of ice-cold tri-fluoro-acetic acid (TFA) to a final pH of 2.5, frozen in liquid nitrogen and stored at -80°C. In the global analysis, incorporation of ²H was measured on PDK1 50-359 [Y288G;Q292A] at a concentration of 2.7 mg/ml (76.5 µM) in the presence and absence of PIFtide (625 µM) and small molecular weight compounds PS48 (500 µM) or PS08 (250µM). For local exchange analysis, similar concentrations of PIFtide, PS48 and PS08 were used. In the presence of peptide or small compounds, protein and ligand solution were pre-incubated on ice for 50 min before the mixture was diluted into labeling buffer. After the exchange reaction, samples were loaded on a pepsin column for one minute for digestion into peptides and samples quenched at each time point. They were then subjected to liquid chromatography for separation and analyzed by tandem mass-spectrometry (LC-MS/MS) for their incorporation of deuterium atoms. The entire plumbing system was immersed in an ice bath. 61 polypeptides were identified with overlapping regions, covering 80 % of PDK1 sequence. As it is not possible to predict the cleavage sites of pepsin in the PDK1 sequence, it was necessary to identify the peptides generated by pepsin digestion for local-exchange analysis. The program Mascot (Matrix Science) was used to assign the peptides from the LC-MS/MS run result, comparing observed m/z value, charge state and

PDK1 sequence.

Isothermal titration colorimetry: Calorimetric titrations were performed using the VP-ITC instrument from MicroCal Inc. (Northampton, MA) as previously described (Schaeffer, F. et al., Biochemistry 41:2106-2114 (2002)) with the following modifications: PDK1 and compounds PS48, PS47, PS08, PS133 were prepared in 50 mM Tris-HCl (pH 7.5), 200 mM NaCl and 1 mM DTT. Titration was performed by 30 successive injections (10 ml) of each compound (450 µM) into a 1.4 ml reaction cell containing PDK1₅₀₋₃₅₉[Tyr288Gly-Gln292Ala] (20 µM). Raw calorimetric data were corrected for heats of dilution. Binding stoichiometries, enthalpy values and K_{d} values were determined by fitting corrected data to a biomolecular model with Origin7 software (MicroCal Inc.).

Protein kinase activity tests: PDK1 activity tests were performed essentially as previously described (Biondi, R. M. et al., Embo. J. 20:4380-4390 (2001); Balendran, A. et al., J. Biol. Chem. 275:20806-20813 (2000); Biondi, R. M. et al., Embo. J. 19:979-988 (2000)) using T308tide as a substrate for PDK1. In brief, PDK1 activity assay was performed at room temperature (22°C) in a 20 µl mix containing 50 mM Tris pH 7.5, 0.05 mg/ml BSA, 0.1% β-mercaptoethanol, 10 mM MgCl₂, 100 µM [γ³²]ATP (5-50 cpm/pmol) , 0.003% Brij, 150 ng PDK1, and

T308tide (from 0.1 to 1 mM). When appropriate, the PDK1 activity assay was performed in a 96 well format and 4µl aliquots spotted with on p8l phosphocellulose papers (Whatmann) using ep motion 5070 (Eppendorf), washed in 0.01% phosphoric acid, dried, and then exposed and analysed using PhosphoImager technology (Typhoon, GE Healthcare). Activity measurements were performed in duplicates with less than 10% difference between pairs. Experiments were repeated at least twice. PDK1 specific activity at 22°C was approximately 0.25 U/mg when the substrate concentration was 0.1 mM T308tide. Since T308tide Km is estimated to be >10mM, the specific activity of PDK1 can be linearly increased with increasing concentrations of T308tide.

Synthesis and characterization of low molecular weight compounds: A full description of the synthesis and characterization of compounds will be published elsewhere (Adriana et al.?).

Probing the conformation of the ATP binding site in PDK1: The activation of PDK1 by HM-polypeptides and small compounds is due to a change in the conformation of the enzyme. We probed the conformation of the ATP binding site in PDK1 by scanning the steady-state fluorescence of TNP-ATP/PDK1, in the presence or absence of P-HM-polypeptides, PIFtide, or small molecular weight compounds. Data were obtained in a Varian Cary Eclipse spectrofluorometer (excitation λ=479nm; emission scanning, γ=500-600nm; excitation slit=10nm; emission slit=10nm) at a rate of 200 nm/min, with 150 datapoints/100 nm scanning and 0.3 s averaging time. The incubation was performed at 20°C in a buffer containing 50 mM Tris pH 7.5, 0.1 mM EDTA, 187 mM NaCl, 40 µM TNP-ATP, 15 µM PDK1 catalytic domain, 1 mM DTT and 1% DMSO. In the absence of PDK1, TNP-ATP produced approximately 1.5 arbitrary units (a.u.) of fluorescence, and the inclusion of PDK1 increased its fluorescence intensity to approximately 3.3 a.u. Inclusion of excess ATP (1 mM) diminished the effect, indicating that ATP competed with TNP-ATP for binding to PDK1, presumably at the ATP binding site. As an additional control, the assays were performed in the absence of PDK1; PIFtide, P-HM-polypeptides and the compounds shown did not modify the TNP-ATP fluorescence in the absence of PDK1. In preliminary tests, inclusion of 2.5 mM MgCl₂ in the assay mix produced similar results. Data for each condition are the average of 3 scans.

Surface Plasmon resonance (BiaCore) and alpha-screen interaction and displacement assays: Binding of GST-PDK1 to a P-HM-polypeptide derived from S6K1 shown in Fig. 6 was analysed by surface plasmon resonance on a BiaCore 3000 system using a streptavidin-coated Sensor chip (SA) and biotin-P-HM-polypeptide, as previously described (Biondi, R. M. et al., Embo. J. 20:4380-4390 (2001). The interaction of His-PDK1 with biotin-PIFtide and the displacement by compounds shown in Fig. 3 was performed using the alpha-screen Ni-Chelate kit (Perkin Elmer) on an Envision 2104 multilabel system (Perkin Elmer).
BiaCore analysis was performed as described previously (Biondi, R. M. et al., Embo. J. 19:979-988 (2000)). PDK1 wt had maximum binding to the chip when PDK1 was injected at 5.4 µM, giving a response of 600RU (Kd=0.5 µM). Since both mutants were performed on the same construct of PDK1, their mass should be very similar and therefore should also produce 600RU when all biotin-(P)HM-S6K polypeptides on the same chip are bound to the mutant PDK1 proteins. Unfortunately, when mutant PDK1 Phe224Trp and PDK1 Thr226Trp were analysed, the injection of the mutants at concentrations higher than 1.3 µM prompted unspecific effects, therefore making difficult the evaluation. However, 1.3 µM and 1.8 µM of the mutant proteins produced more than 200RUs of binding, indicating that they bound the phosphorylated HM polypeptide derived from S6K with high affinity. We further verified that the binding of mutants to the chip was specific (due to binding to the PIF-binding pocket on PDK1) since we displaced the binding of the PDK1 CD proteins to the chip when the proteins were preincubated with PIFtide previous to injection.

The interaction between His-PDK1 50-556 and biotin-PIFtide was assayed using the Ni-Chelate kit from Perkin-Elmer in an Envision system. To study if low molecular weight compounds displaced the interaction, the compounds were incubated with the mix and the displacement of the His-PDK1 biotin-PIFtide interaction measured as a decrease in light emission.

**Table I: Data collection and refinement statistics**

| Dataset | PDK1*dm* + atp | PDK1*dm* + atp + ps48 |
|---|---|---|
| *A. Data collection* | | |
| Beamline (ESRF) | ID14-1 | ID23-1 |
| Wavelenght (Å) | 0.9340 | 0.9791 |
| Space group | C2 | C2 |
| Unit cell dimensions | | |
| a (Å) | 148.08 | 148.55 |
| b (Å) | 43.79 | 44.00 |
| c (Å) | 47.49 | 47.24 |
| β (α, γ = 90°) | 101.50 | 100.42 |
| Resolution (Å) | 46.5-1.9 (2.0-1.9) | 46.5-1.9 (2.0-1.9) |
| Unique reflections | 22,445 | 23,875 |
| Completeness (%) | 96.4 (80.4) | 99.8 (100) |
| Multiplicity | 3.7 (3.2) | 3.3 (3.3) |
| Mean (I / σ (I)) | 15.3 (3.6) | 10.1 (3.9) |
| R_{merge} | 0.064 (0.315) | 0.094 (0.368) |
| | | |
| *B. Model refinement* | | |
| Reflections used | 22,082 | 23,490 |
| R-factor | 0.174 | 0.153 |
| R_{free} | 0.213 | 0.180 |
| Refined non-H protein | | |
| atoms | 2248 | 2241 |
| Refined solvent atoms | 179 | 199 |
| r.m.s.d. from ideal | | |
| Bond lengths (Å) | 0.026 | 0.028 |
| Bond angles (deg) | 2.211 | 2.370 |
| Mean B-values (Å²) | 14.011 | 22.213 |

### Example 1

A. Crystallization of PDK1 in a novel crystal packing: In order to understand the molecular details of the interaction between PDK1 and low molecular weight compounds activators of PDK1, we decided to obtain crystallographic information of the compound-PDK1 complex. We expressed and purified PDK1 50-359 (Engel, M., Embo. J. 25:5469-5480 (2006)), and further reproduced conditions which led to diffracting crystals in the past (Biondi, R. M. et al., Embo. J. 21:4219-4228 (2002)). However, upon extensive robotic screening of crystallization conditions we found that PDK1 50-359 only crystallized in one crystal packing and that small compounds could not be soaked or co-crystallized with PDK1 50-359 in such packing (see Supplementary Materials and Methods). We reasoned that the occupancy of the PIF-binding pocket by Tyr288 from a neighboring subunit in the crystal could impede the binding of compounds to the PIF-pocket within the crystal. We therefore analysed the possibility to crystallize PDK1 and PDK1-compound complexes using a set of mutants of PDK1 disrupted in the crystallographic contacts along the PIF-binding pocket. PDK1 50-359[Tyr288Gly; Gln292Ala] crystallized in a different crystal packing (now termed crystal packing II), that exposed the PIF-binding pocket.

The structure of PDK1 in the crystal form II in the absence of compound was solved by molecular replacement using the PDK1 1H1W structure (Biondi, R. M. et al., Embo. J. 21:4219-4228 (2002)) as a model and refined to 1.9 Å (R-factor of 0.17 and an R_{free} of 0.21; Table I). Superimposition of the structure of the native PDK1 with the structure of the mutant PDK1[Y288A-Q292G] showed that the conformation of PDK1 remained very similar. The secondary structures in both small and large lobes almost perfectly superimposed the equivalent ones in the original protein structure (Fig. 1A), although there was a significant movement of the small lobe relatively to the large lobe. Upon fixing the large lobe residues, the RMS deviation between the native form and the crystal form II on the small lobe Cα atoms was 1.18 Å. In spite of the global similarity, some differences in the secondary structures could be seen in the small lobe, affecting the glycine-rich loop, the PIF-binding pocket (α-B and α-C helices, indicated in Fig. 1B) and the phosphate binding site (described below).

Within the key regulatory HM/PIF-binding pocket region, the HM-phosphate binding site was found to be disrupted in crystal packing II since residues defining the phosphate binding site were not visible (Lys76) or were in a different conformation, rotated 90° (Arg131). This was likely due to the lack of sulphate in the crystallization condition which positioned the residues in a phosphate-binding competent conformation in the crystal packing I. On the other hand, an ordered water molecule in crystal packing II occupied the place of the sulphate ion and coordinated the two other residues, Thr148 and Gln150, which were positioned as in the original PDK1 structure. The HM binding region within the HM/PIF-binding pocket appeared otherwise similar to that observed in the crystal packing I, where Phe157 points towards the inside of the PIF-binding pocket, generating a shallow pocket that would preclude the binding of HM polypeptides.

B. PDK1 complexed to PS48. Binding mode and effects on the PIF-binding pocket: The crystal structure of PDK1 bound to low molecular weight compounds was obtained from co-crystallization trials set in the presence of ATP and low molecular weight compounds and also by soaking experiments on crystal packing II, with similar results. The model structure of the PDK1 complex with ATP and a low molecular weight compound termed PS48 (obtained from a crystal soaked with PS48) was solved to 1.9 Å resolution (R-factor 0.15; Rfree 0.18; Table I). PS48 was synthesized by us as part of a focused library of compounds directed to the PIF-binding pocket and identified as an activator of PDK1 (see the PS48 formulae in Fig.2A). The PS48 compound class was of particular interest because the ease of synthesis allowed us to produce a number of high quality pure compound analogues -without racemic mixtures- which could help us understand the requirements for the activation of PDK1 by low molecular weight compounds. The synthesis, characterization and the full structure activity relationship data obtained from forty PS48 analogues will be published separately. The overall structure in the presence of compound differed only slightly from the apoPDK1 structure described above (Fig. 2B), mainly within the PIF-binding pocket and its associated phosphate-binding site, the ATP binding site and the activation loop. From early refinement steps, a strong peak appeared in the |Fo-Fc| difference map, in the region of the PIF-pocket. Our compound scaffold could be assigned to this density, most notably the two phenyl rings and the carboxylic side chain. The well-defined electron density for the compound at a level of 1.0 σ (Suppl. fig 1) was consistent with high occupancy and the presence of a single ligand conformation. The accessible surface area buried by the interaction was 240 Å². The mode of interaction is reminiscent of the mode of binding of PKA C-terminus to its own pocket (Knighton, D. R. et al., Science 253:407-414 (1991)) (Suppl Fig. 2). Remarkably, the phenyl rings in PS48 mimicked the positioning of the two Phe aromatic side chains, each occupying one of the two sub-pockets like it is seen in PKA. As a major difference to all other PDK1 crystal structures described so far, the low molecular weight compound induced an important movement on Phe157, which is found in a different conformation, pointing inwards instead of occupying the base of the pocket (Fig. 2C and 2D). The conformational change enlarged the depth of the pocket and allowed the ring closer to the carboxylate group from PS48 to enter the pocket. In the presence of PS48, Lys76 and Arg131 move to interact with the carboxylate from PS48 (Fig. 2E and 2F). Interestingly, interactions between PS48 carboxylate and the surrounding charged and polar side chains in the PIF-binding pocket were similar to the interactions observed by the sulphate ion with Lys76, Thr148, Gln150 and Arg131 in crystal packing I (Fig. 2F). These data are in agreement with our previous biochemical characterizations that highlighted Arg131 positive charge as a key partner for activation by low molecular weight compounds (Engel, M., Embo. J. 25:5469-5480 (2006)). In the presence of PS48, all these surface residues had decreased B-factors, altogether indicating a dynamic rearrangement of the surface residues to complement and stabilize the bound compound. In addition, Lys115, also had lower B-factors in the presence of PS48, possibly due to hydrophobic interactions with the compound and interaction with Gln150 which is stabilized by the PS48 carboxylate via a water molecule. Altogether, the crystallography derived structural data confirmed that the small activating compound PS48 indeed bound to the PIF-binding pocket. In addition, the data provided crystallographic evidence for the stabilization of the putative phosphate binding site residues by the interaction with the carboxylate from PS48. This result reinforced the idea that the carboxylate from small compounds binds to residues forming part of the phosphate-binding site. The structure suggests that the mirror image isomer of PS48 and its analogues would not bind to PDK1. In agreement with this hypothesis binding of the PS47 and PS133 (the isomers respectively of PS48 and PS08) was not detected in ITC experiments, nor did they displace the binding of PIFtide to PDK1 (see below), nor did they activate PDK1 (Table II). These results indicated that the crystal structure data fitted well with the studies in solution. Furthermore, the results demonstrated that the data obtained for the crystal structure of a mutant form of the catalytic domain of PDK1 also applied to the full length PDK1 protein. Most notably, the biochemical data supported the positioning and interactions of the carboxylate with the potentially most conformation-variable residues surrounding the PIF-binding pocket, Lys115, Lys76, Gln150 and Arg131.

The binding to PDK1 was not detected in ITC experiments (see below), nor did they activate PDK1 or displaced PIF binding in alpha-screen studies.

C. PDK1 complexed to PS48. Effects on the ATP binding site and activation loop: In the presence of PS48the bound nucleotide adopts the same conformations in the apo-structure. However, we observed a movement of Lys111 which approached α-C-helix residue Glu130. This displacement can be due to the conformational change of the PIF-binding pocket Phe157 residue described above, as (Cγ and Cδ atoms) the aliphatic chain of Lys111 formed hydrophobic interactions with (Cδ1 and Cε1) the aromatic ring of Phe157. Since it is well established that the equivalent Lys-Glu pair coordinates the ATP molecule in other protein kinases, the small but significant movements of both residues (distance of 3.1 Å in the apo PDK1 structure and 2.7 Å in the complex) could mediate an allosteric effect on the ATP binding site upon binding of compounds to the PIF-binding pocket. Compared to the apo structure, in the complex with PS48, there is also a significant change at the top of the ATP binding site, along the glycine-rich loop. Here, the side chain of Phe93 rotated approximately 110° (χ1 angle = -60° versus +53°) suggesting that a glycine-rich loop residue can also transduce allosteric effects from the PIF-binding pocket to the ATP binding site (Fig. 2G and 2H). Phe93 conformation is also affected upon binding of ATP competitive inhibitors such as staurosporine, and thus, the glycine-rich loop appears as a sensor of both PIF-binding pocket and ATP binding site occupancy by activators and inhibitors.

However, perhaps the most striking overall feature that differs between the apo and PS48 complex structure lied in the ordering of the activation loop in the presence of compound (Fig. 2G and 2H). In the apoPDK1 structure, residues 233 to 236 were disordered. In addition to ordering of the activation loop, there was also some degree of movement. Thus, in the apoPDK1 the amine group of Lys228 and phospho-Ser241 were at a distance of 2.9 Å whereas in the complex they were 4 Å apart. Lys228 amine group displacement (1.5 Å) also triggered a new interaction with Arg129 via an ordered water molecule (<B-factor> = 36.9 Å²) that was absent in apoPDK1. The new location of Lys228 also enabled it to interact with Gln236 from the activation loop via another water molecule (<B-factor> = 29.6 Å²) (Fig. 2H). Overall, the data showed that the binding of PS48 allosterically affected the ATP binding site and the activation loop.

D. Characterization of the binding properties of compounds of the ps48 family to

PDK1: In order to characterize the binding of compounds to PDK1 we performed experiments with the PDK1 catalytic domain protein used for crystallization studies (PDK1 50-359 [Y288A-Q292G]) and also with the PDK1 50-596 protein. The binding properties of PS48 and its mirror image compound PS47 isomer to PDK1 50-359 [Y288A-Q292G] were determined by isothermal titration calorimetry (ITC) at 20°C (Fig. 3A; Table IIIa). PS48 bound to PDK1 50-359 with a 1:1 stoichiometry and a binding affinity in the micromolar range (*K*_{d} = 6.2 µM). In sharp contrast, under similar conditions, the mirror image compound PS47 did not bind to PDK1 at any significant level (Fig. 3A). Similarly to PS48, its analogue compound PS08, but not its mirror image compound PS133, bound to PDK1. Binding of compounds PS48 and PS08 was both enthalpy and entropy favorable and driven by the entropy term (DH/DG = 20.5% and 24.1%, respectively; Table III). Such binding characteristic is a general property of hydrophobic interactions which is consistent with the burial of the phenyl rings from PS48 and PS08 in the hydrophobic PIF-binding pocket.

Similarly, using alpha-screen technology, we could verify that the binding of PIFtide to PDK1 protein containing the C-terminal PH domain (50-556) could be displaced by PS48 and PS08 but not by their isomers PS47 and PS133 (Fig. 3B). Altogether, the data suggested that the lack of activation of mirror image compounds PS47 and PS133 was due to lack of binding to PDK1.

E. Probing the conformational change induced by HM-polypeptides and allosteric compounds by use of the fluorescent ATP analogue TNP-ATP: In order to shed further light on the conformational change induced by the binding of small compounds to the PIF-binding pocket of PDK1, we developed a method to probe the ATP binding site conformation in solution with the use of a fluorescent ATP analogue, trinitrophenyl-ATP (TNP-ATP; Fig. 4A), as detailed under Experimental procedures. The fluorescence intensity of TNP-ATP increased with the addition of PDK1 (Fig. 4B) and was competed with the addition of excess of ATP, indicating that the TNP-ATP fluorescence intensity increased upon binding to the ATP binding site on PDK1. Interestingly, the TNP-ATP fluorescence intensity decreased with the subsequent addition of PIFtide or phosphorylated HM polypeptides (not shown). Similarly, low molecular weight compound activators of PDK1, represented by PS08, produced a concentration-dependent decrease in TNP-ATP fluorescence in the presence of PDK1 (Fig. 4C), while they had no effect on TNP-ATP fluorescence in the absence of the kinase. In the same assay, the mirror image / enantiomer compound of PS08, PS133, did not affect the fluorescence intensity of TNP-ATP in the presence of PDK1, indicating that the effect produced by PS08 was highly specific (Fig. 4D). Altogether, the results showed that the binding of activating molecules targeting the PIF-binding pocket of PDK1 produced a significant allosteric effect which could be sensed at the ATP-binding site. Since the decrease in fluorescence intensity was also measured with phosphorylated HM polypeptides and

PIFtide, it is likely that the ATP-binding site conformational change measured was actually related to the equivalent events that activate PDK1 by low molecular weight compounds and HM-polypeptides.

F. Allosteric effects of small compounds upon binding to the PIF-binding pocket on PDK1 revealed by amide hydrogen/deuterium (¹H/²H) exchange and mass spectrometry: We also analysed the effect of compound binding and the possible conformational changes induced by polypeptides and small compounds on PDK1 by studying their ability to protect or expose regions of the protein to hydrogen/deuterium (¹H/²H) exchange. PDK1 50-359[Tyr288Gly; Gln292Ala] was incubated in the presence or absence of PIFtide or small compounds in buffer containing ²H ₂O for different incubation times, the exchange process was stopped with addition of TFA and incubation on ice and the PDK1 mass evaluated by mass-spectrometry. The mass of PDK1 increased along the time of incubation in ²H ₂O buffer indicating that ²H was incorporated into the protein backbone amide group. The incubation of PDK1 with the polypeptide PIFtide (0.625 mM) prompted a significantly lower increase in PDK1 mass along time, equivalent to 20 ²H less incorporated / PDK1 molecule after 45 minutes, indicating a large protection on the overall ¹H/²H exchange. A similar global exchange protection was also observed when PDK1 was incubated with PS48 (500µM) or PS08 (250µM), although to a lower degree (10-12 ²H / PDK1 molecule). Under the conditions tested, only the backbone amide group can exchange its ¹H for ²H, giving rise to a maximum of one ²H exchanged per amino acid (except proline). Thus, the global exchange analysis suggested that low molecular weight compounds activators protected between 10 and 12 amino acids from ²H exchange after 45 minutes incubation.

In order to identify the PDK1 sequences that were affected by the interaction of compounds to the PIF-binding pocket, we repeated the ¹H/²H exchange experiments at different incubation times, digested PDK1 at each time point with pepsin and subjected the polypeptides to liquid chromatography followed by tandem mass-spectrometry (LC-MS/MS). After pepsin digestion, we could identify and analyse 36 polypeptides with overlapping regions (see Materials and Methods), covering 84.5% of PDK1 50-359[Tyr288Gly; Gln292Ala] sequence. In the presence of low molecular weight compounds, a strong protection was observed in peptides corresponding to the amino acid sequences 108-121 (Fig. 5B), 108-134 (Fig. 5C),108-130 (not shown), covering the α-B and α-C helices and 146-155 (Fig 5D) spanning the β-5 strand, all limiting the PIF-binding pocket (Fig. 5A). The polypeptide 122-134, corresponding specifically to the α-C-helix was very strongly protected by PIFtide, especially at early time points (a protection of five ²H at 1min, see Supplementary Fig. 3), but was only weakly protected by PS08 (Fig. 5E) (a protection of one ²H after 1 minute and protection was not significant thereafter) and PS48 (not shown), suggesting that the α-C-helix protection had very different ¹H/²H exchange kinetics to other polypeptides. The results support the idea that the compounds indeed bound to the PIF-pocket in solution, and that, within the pocket, the strongest protection was observed on the α-B-helix.

In addition to the protection at the PIF-binding pocket, we observed protection from ¹H/²H exchange at sites distinct from the compound binding site. Thus, peptides 49-93 (Fig. 5F) and 52-93 (Fig. 5G), but not the shorter peptide 49-66 (Fig. 5H) were strongly protected by low molecular weight compounds. The protected amino acid sequence (67-93) spans the β-1 strand and Gly-rich loop, at the top of the ATP binding site. This finding correlates well with the conformational change of Gly-rich loop Phe93 observed in the crystal.

As expected, the polypeptides spanning the activation loop (218-247 and 225-247;

Fig. 5I and J) readily exchanged ¹H for ²H with most protection on 218-247, suggesting that the most significant protection occurred between amino acid 218 and 224. Interestingly, this region does not correspond to the activation loop but rather to the β-8 sheet and up to the DFG motif (Phe224) located within the ATP binding site. The above results allow to pin-point the regions corresponding to the Gly-rich loop and β-8 / DFG motif within the ATP binding site as key sites allosterically protected from ¹H/²H exchange by low molecular weight compounds in solution.

Unexpectedly, all low molecular weight compounds tested also triggered protection from ¹H / ²H exchange at a distal region, on three polypeptides (276-291, 292-311 and 292-316) spanning the α-G-helix within the large lobe of the catalytic domain (see Supplementary Fig. 4). Interestingly, the overlapping peptides (298-311 and 302-311) were not protected in the presence of compounds, thus defining the region undergoing the protection between residues 292 and 298 in the α-G-helix. However, this conformational change is unlikely to be responsible for the activation of the kinase because it was not observed when the protein was incubated with PIFtide.

Altogether, the results from the ¹H / ²H exchange experiments supported the biochemical and crystallographic identification of the PIF-binding pocket as the target of the small compounds. In addition, the data provided support for the crystallographic and fluorescent data that suggested that the ATP binding site was a target of the allosteric effect prompted by the binding of low molecular weight compounds to PIF-binding pocket. Finally, the ¹H / ²H exchange data uncovered a further allosteric effect on the α-G-helix on the large lobe of the kinase, suggesting that binding of small compounds to the PIF-binding pocket can prompt conformational changes unrelated to the activation of the catalytic domain.

G. Mutation at Thr226 in PDK1 uncouples the ligand binding from activation: In order to shed light on the molecular requirements for the allosteric transition, we generated PDK1 proteins (50-359) mutated in a Phe or Thr residue located within or just next to the DFG motif. In the first mutant, the Phe residue from the DFG motifi was mutated to Trp (PDK1[Phe224Trp]). In the second mutant, the Thr residue within the sequence DFGT was mutated to Trp (PDK1[Thr226Trp]). We did not expect that the Thr to Trp mutation would greatly disrupt the functioning of the protein since most AGC kinases posses a hydrophobic Phe residue at this position. We first tested the effect of the mutations on PDK1 intrinsic activity. Activity tests indicated that both PDK1[Phe224Trp] and PDK1[Thr226Trp] mutants had 3 fold reduced specific activity as compared to the wild type protein, indicating that they were overall well folded. Further biochemical characterization revealed that the wild type protein and PDK1[Phe224Trp] were activated by PIFtide. In sharp contrast, PDK1[Thr226Trp] was not activated by PIFtide (Fig. 6A). In support to these findings, when the mutations were performed on the GST-PDK1 1-556 protein, the corresponding mutant proteins behaved similarly to the catalytic domain counterparts described above. The lack of activation of PDK1[Thr226Trp] could be explained by a lack of binding of the mutant protein to PIFtide.

Surprisingly, however, surface plasmon resonance experiments indicated that the wild type and mutant proteins bound to biotin-PIFtide and biotin-P-HM-S6K to similar levels (approximately 50% of maximal binding of the wild type protein). Thus, when injected at similar concentrations (e.g. 1.8 vs 1.3 µM) both mutant proteins bound about 200 response units to the chip (Fig. 6D and E). In spite of the unspecific binding to the chip observed in both mutants when tested at higher concentrations, PIFtide displaced the binding of both mutants at similar concentrations, suggesting that the affinities to PIFtide were similar between these mutants. These studies indicated that PDK1[Phe224Trp] and PDK1[Thr226Trp] bound the HM polypeptides with similar affinities to the corresponding non mutated PDK1 counterpart. Thus, the above data showed that PDK1[Thr226Trp] was able to bind PIFtide but was not activated by this interaction. The above results suggested that mutation of Thr226 in PDK1 does not abolish activity but abolishes the activation by PIFtide, defining a region at the entrance to the ATP binding site and directly interacting with the α-C-helix as a site which uncouples the binding of PIFtide from the activation mechanism.

H. Unsuccessful crystallization of PDK1 50-359 wild type construct in complex with compounds: In order to get PDK1-compound complexes, crystals of wild-type PDK1 were grown in the presence of various nucleotides and a selection of about 30 compounds which had been found to be active in solution assays. In addition, several high throughput screenings for crystallization conditions in the presence of compounds were performed. Crystallization screens selected were Crystal Screens 1 & 2, PEGion, MembFac, Natrix, SaltRx and Cryo (Hampton Research), the Structure Screens 1 & 2 (Molecular Dimensions Ltd), the JBScreen1-4 and 5-8 (Jena Bioscience) and the Wizard 1 & 2 screens (Emerald Biosystems). Although over 3800 conditions were tested, crystals appeared only in the condition, similar to that previously identified (Biondi, R. M. et al., Embo. J. 21:4219-4228 (2002)). However, crystals were not obtained in the presence of compounds.

In order to obtain complexes with compounds, we also explored soaking crystals of PDK1 50-359 in crystal packing I with low molecular weight compound activators. Soaking of PDK1 50-359 crystals (in crystal packing I) with compounds prompted dissolution of the crystals. Five PDK1-ATP crystals soaked with compounds were frozen before dissolution of the crystals and data were collected in-house or at the ESRF beam line ID14-EH3. All four datasets were indexed in the same trigonal space group P3₂21 as the PDK1-ATP structure in crystal packing I (Biondi, R. M. et al., Embo. J. 21:4219-4228 (2002); PDB: 1H1W). Data were processed and the structures refined (see Methods). All five electron density maps showed that the PIF-binding pocket was occupied by the side-chain of Tyr288 in the αG-helix from a neighbouring molecule. Thus, after extensive testing, we concluded that it may not be possible to obtain PDK1 complexed to low molecular weight compounds activators using the wild type PDK1 50-359 construct, which only crystallized in crystal packing I.

I. Mutation at Thr226 in PDK1 uncouples the ligand binding to the HM/PIF-pocket from activation of the kinase: To further verify that the binding of PDK1[Phe224Trp] and PDK1[Thr226Trp] to P-HM-S6K was indeed specific and with a similar affinity to the wild type protein, we pre-incubated the PDK1 proteins with distinct concentrations of PIFtide previous to injection. In this competition experiment, PIFtide would interact with the PDK1 protein and this would be measured as a displacement from PDK1 binding to biotin-P-HM-S6K. If the bulk of PDK1[Phe224Trp] and PDK1[Thr226Trp] was specifically mediated by the HM/PIF-pocket, the binding to the biotin-P-HM-S6K chip would be displaced by PIFtide. Furthermore, if similar affinities were involved, the displacement of the binding would take place at similar concentrations of PIFtide. In contrast, if the binding was not due to its interaction with the HM/PIF-pocket in PDK1, PIFtide would not displace this interaction Indeed, the binding-competition experiments showed that similar concentrations of PIFtide displaced the binding of the three proteins to biotin-P-HM-S6K. These studies clearly indicated that PDK1[Phe224Trp] and

PDK1[Thr226Trp] bound the HM polypeptides with similar affinities to the corresponding non mutated PDK1 counterpart.

Altogether, the data indicated that PDK1 Thr226Trp bound PIFtide, but was not activated, suggesting that Thr226 environment (between the α-C-helix and the ATP binding site) participated in transducing the binding of PIFtide to the conformational change that activates PDK1.

J. Deuterium exchange: Unexpectedly, all the low molecular weight compounds tested also triggered protection of 1H / 2H exchange at a distal region, spanning the α-G-helix within the large lobe of the catalytic domain. Thus, polypeptides 292-311, 292-316 and 295-311 showed a significant protection along the incubation time, whereas the polypeptide 298-311 and 302-311 lacked all protection at any time point. Thus, we concluded that the amino acids protected from ¹H/²H exchange must be within residues 292 and 297, which form part of the α-G-helix on PDK1. We were very confident on the data indicating the protection of the 292-297 site because the data was very reproducible, on different overlapping polypeptides and present at different concentrations of compounds. In addition, all the above results were verified with a ten times more potent compound from a different class (not shown).

Also observed was a significant protection by PIFtide on overlapping polypeptides corresponding the amino acids 193-212, 194-210, 194-212 and 194-213. This protection was not evident after incubation with PS48, but was significant over three points along the time course with PS08, a more potent compound.

**Table 3: Thermodynamic parameters for binding of isomer compounds ps48/ps47 and test8/ps133 to PDK1₅₀₋₃₅₉ at 20°C determined by ITC.**

| | | N | *K*ₐ (M ⁻¹) | *K*_{d} (µm) | Δ*H* cal/mol | Δ*G* cal/mol | TΔ*S* cal/mol | Δ*H*/ Δ*G* |
|---|---|---|---|---|---|---|---|---|
| **PS48** | 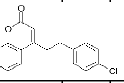 | 1.01 | 1.61E5 | 6.2 | -1,439 | -7,029 | 5,590 | 20.5% |
| **PS47** | 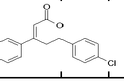 | - | - | - | - | - | - | - |
| | | | | | | | | |

| | | N | *K*ₐ (M ⁻¹) | *K*_{d} (µm) | Δ*H* cal/mol | Δ*G* cal/mol | TΔ*S* cal/mol | Δ*H*/ Δ*G* |
|---|---|---|---|---|---|---|---|---|
| **PS08** | Z | 1.01 | 1.64E5 | 6.1 | -1,700 | -7,040 | 5,306 | 24.1% |
| **PS133** | E | - | - | - | - | - | - | - |

### Example 2

### A. The double mutant PDK1[Y288G-Q292A] crystallized in two other crystal forms:

In an attempt to fully characterize the structural changes brought about by the allosteric transition, we performed a number of high throughput screenings for crystallization conditions of PDK1[Y288G-Q292A] in the presence of HM-peptides. Crystals of PDK1 in the presence of PIFtide and ATP were obtained with 2 M ammonium sulfate and 0.1 M sodium citrate pH 5.6. They had a different morphology to the other two PDK1 crystal packing forms (crystal packing I and crystal packing II) as they looked like very thin plates. It was possible to collect diffraction data at the ESRF ID23-1 beam line to 3.2 Å. These crystals corresponded to a new crystal form, called crystal form III, in the monoclinic space group C2. The structure was solved by molecular replacement using the original PDK1 structure.

In parallel, it was tried to improve the crystals quality to get better data. To this end, crystals were reproduced by refining the mother liquor conditions, and the substrates to co-crystallize: the HM peptide and the nucleotide. I obtained larger crystals in the same condition as mentioned above in the presence of different HM-peptides and the ATP analogues AMP-PCP and AMP-PNP. Datasets were collected at ESRF beam lines ID14-2 and ID14-3. Surprisingly, these crystals appeared as a different crystal form as the previous ones. Cell parameters were extremely close to the crystal form III as they are identical except for an inversion of cell dimensions a and b (Table 4). However, the space group was different as the new datasets indexed in the monoclinic space group P2(1). Therefore, we named the latter crystal form, form IV.

The preliminary study of electron density maps in both crystal forms showed very interesting features that differ from the two other crystal structures of PDK1 obtained from crystal packing I and II. In particular, the nucleotide molecule in the catalytic site appeared in a different conformation, closer to the functional conformation observed in other kinases. Finally, although the HM-peptide could not be modeled in most structures of both forms, one structure allowed to partially model the peptide, providing for the first time structural information on the interaction between PDK1 and a HM-peptide substrate.

B. The crystal forms III and IV: It was striking to obtain two different crystal forms in a condition that was basically the same. We assumed that the obtention of two forms was due to the presence of different HM-peptides and nucleotide in the co-crystallization mixture. Two structures, one of each form, were selected on the basis of the data statistics to be compared.

To represent the crystal form III, the structure selected was PDK1_30, which crystal grew in the presence of ATP and PIFtide (REPRILSEEEQEMFRDFDYIADWC). The structure in the form IV that was obtained to highest resolution was PDK1_33. The corresponding crystals were grown in the presence of AMP-PNP and the HM-peptide (KGAGGGGFPQFSYSA) derived from PKB sequence.

The major difference between both packings was that in the form III (space group C2) the asymmetric unit (ASU) contained two molecules whereas the ASU contained four molecules in the form IV (space group P2(1)). We superimposed two chains of PDK1_33 with the crystallographic dimer in PDK1_30 and generated the symmetric dimer of the PDK1_30 dimer (Fig. 7). The four resulting molecules in PDK1_30 and the four molecules in PDK1_33 ASU could be superimposed suggesting that there was a slight difference between both crystal forms.

Although the forms III and IV looked highly similar, there was no doubt on the space group attribution. Therefore, it was clear that form IV lost one crystallographic symmetry operator in comparison to the crystal form III, i.e. a global (crystallographic) symmetry operator became a local (non-crystallographic) one. In order to further study the crystal contacts between the two structures and identify which contacts may be responsible for the change, we superimposed both structures and studied all intermolecular contacts in both structures. Despite a careful study of the interfaces between the molecules in both structures, there was no contact that appeared to be different between both structures. However, there was a movement of the four molecules forming the crystallographic tetramer in the crystal form IV that push them slightly away from each other (Fig. 8). This displacement was sufficient to lose a crystallographic symmetry between one dimer and the other. As no crystal grew in the absence of peptide, we assumed that the peptides were present between the molecules and that the difference in length between the two peptides could be the reason for the displacement. Indeed, PIFtide being nine amino-acids longer than the PKB-HM derived peptide, it could be hypothesized that the occupation of PIFtide between the molecules in the crystal would push the cluster of molecules, in the middle, to closer contacts.

C. General remarks on the new structures: The RMS on Cα atoms deviation between a PDK1_30 molecule and a PDK1_33 molecule is about 0.3 Å. Both structures PDK1_30 and PDK1_33 have a similar RMS deviation on the Cα atoms to the original PDK1 structure (0.45 and 0.43 Å respectively) and to the crystal form II structures (0. 40 and 0.33 Å). The figure 9 shows the main-chain and side-chain RMS deviations between the new crystal forms and to the crystal form II. The larger deviations are in the small lobe and in the activation loop that is disordered.

In both forms, the tip of the activation loop appeared disordered. Residues from Pro232 to Gln236 could not be modeled in PDK1_33 and from Pro232 to Val243 in PDK1_30.

Secondly, there was clear density for the nucleotides in all the structures obtained. Interestingly, the ATP in the structure PDK1_30 was in a conformation that is different from that seen in previous PDK1 structures. The conformation of the ATP molecule in PDK1_30 will be described in more details in this chapter. The AMP-PNP molecule in PDK1_33, appears to have a disordered or degraded γ-phosphate, since only the α- and β-phosphate are seen in the density. It is likely that the γ-phosphate was degraded as AMP-PNP is known to be a rather unstable ATP analogue.

Finally, in PDK1_30, PIFtide could not be modeled. This may be due to the 3.2 Å resolution of this dataset or to a low molar ratio between the protein that was highly concentrated and the peptide that could not be kept soluble at high concentration. Another explanation could be that PIFtide was not completely ordered. In contrast, there was density at the PIF-pocket site in PDK1_33 and the peptide derived from PKB-HM could be partially modeled. In addition, the conformation of the Phe157 side-chain, lying against the bottom of the pocket, was compatible with the binding of the phenyl rings in the PIF-pocket. Importantly, this was the first structure of PDK1 obtained in the presence of a HM-peptide.

D. PKB-HM peptide is present in PDK1 33: Upon refinement of PDK1_33, the electron density map showed density in the PIF-pocket region. This density allowed partial modelling of the PKB-HM peptide. As shown in the Figure 10 the peptides faced each other in the crystal. As the peptide in PDK1_30 was longer than in PDK1_33, this may explain why the four molecules in PDK1_30 appeared closer together.

The PKB-HM peptide was first modeled as an Alanine hexapeptide. Along refinement, density for some side-chains appeared, such as the conserved Tyr residue of the HM. Further on, density for the conserved Phe side-chains could be seen. However, the map around the peptide was not of very good quality so we decided not to model the side-chains of these residues. This could be explained by a rather low molecular ratio between the peptide and the protein that could lead to a partial occupancy of the site in the crystal. Interestingly, although the peptide was not phosphorylated, density appeared in the PDK1_33 map at the site where the phosphate from a phosphorylated HM would stand. In comparison to PKB[S474D] (Yang, J, et al., Mol. Cell. 9(6): 1227-1240 (2002)), this density was located in the place of the Asp474 side-chain. It was not clear upon assignment of this density whether it corresponded to water molecules or to a molecule of sulfate which is a component of the mother liquor.

E. In the crystal form III, the ATP molecule adopted a conformation that resembles the "closed" PKA structure: The most striking features in the PDK1_30 structure were the conformations of the ATP molecule and of the Tyr126 residue in the αC-helix. The density map of PDK1_30, at 2 σ level, showed very clearly that the ATP conformation was distinct from previous PDK1 structures (Fig. 11). The conformation of the ATP molecule appeared similar to the ATP conformation in the "closed" PKA structure as the adenosine and the α- and β-phosphates superimposed the equivalent ones in PKA. However, the γ-phosphate pointed in the opposite direction than the ATP molecule in PKA (Zheng, J et al., Biochemistry 32(9): 2154-2161 (1993)) or the AMP-PNP in PKBβ (Yang, J, et al., Mol. Cell. 9(6):1227-1240 (2002)).

A possible reason for the mispositioning of γ-phosphate may be the absence of divalent cations. The divalent cations Mg²⁺ and Mn²⁺ allow the ATP phosphate groups to form a kink between the β- and γ-phosphates (Johnson, DA et al., Chem. Rev. 101(8):2243-2270 (2001)). Furthermore, the positively charged ions mediate otherwise unfavorable electrostatic contacts of the negatively charged phosphate groups with the negatively charged Asp from the DFG motif. In the PDK1-ATP structures, except for PDK1_30, the phosphate groups are present in an extended conformation, and absence of the Mg²⁺ ions results in loss of the conserved ionmediated contacts because of charge repulsion.

Another difference is that both PKA-ATP/Mg²⁺ and PKBβ-AMP-PNP/Mn²⁺ were co-crystallized with a substrate peptide, which may have stabilized a proper orientation of the γ-phosphate. However, other protein kinases, such as p38α MAP kinase or casein kinase-2 (CK2), have been co-crystallised with Mg²⁺ and AMP-PNP in the absence of substrate peptide (Bellon, S et al., Structure 7(9):1057-1065 (1999); Niefind, K et al., EMBO. J. 17(9):2451-2462 (1998)), and display two Mg²⁺ ions and a conformation of AMP-PNP in which the γ-phosphate resembles that observed in the PKA and PKBβ structures.

Another difference was observed at the ATP binding site as the Gly-rich loop is more closed in PDK1_30 and PDK1_33 than in other PDK1 structures. As the Gly-rich loop conformation has been demonstrated to be characteristic of the activation state in kinases (Johnson, DA et al., Chem. Rev. 101(8):2243-2270 (2001)), this may be of importance in the analysis of this structure in the context of the activation mechanism of PDK1.

Interestingly, the Tyr126 appeared in a completely different conformation than in other PDK1 structures as it points towards the β-phosphate group of the nucleotide. This conformation was not seen in other kinases structures to our knowledge except for the equivalent residue in the atypical PKCτ, Trp289, which appeared in a similar position (Messerschmidt et al, 2005, PDB: 1ZRZ). However, no analysis of the role of this conformation for activity could be carried out as the structure was bound to the bis(indolyl)maleimide inhibitor BIM1 in the ATP site.

The equivalent residue in PKA, His87, is crucial for activity. In fact, His87 in the αC-helix, is the only link between the HM binding site and the activation loop site. In PDK1, Tyr126 and Arg129 link the PIF-pocket to the activation loop site.

In order to identify the importance of Tyr126 for activity, (Komander, D. et al., J. Biol. Chem. 280:18797-18802 (2005)) mutated Tyr126 to Ala. The aim was then to determine the role of the interaction of the Tyr126 side-chain with the phosphate of Ser241 as seen in the crystal form I (Biondi, R. M. et al., Embo. J. 21:4219-4228 (2002), PDB: 1H1W). The experiments showed that PDK1[Y126A] possessed similar basal activity to wild-type PDK1. Activation by HM-PRK2 peptide was impaired in the mutant enzyme as it was activated only 2-fold instead of 5-fold in the wild-type enzyme. This result demonstrated the importance of Tyr126 for PDK1 activation mechanism that could be explained by the interaction with phospho-Ser241. Komander, D. et al., J. Biol. Chem. 280:18797-18802 (2005), in addition, suggested that the Tyr126 mutant still bound PIFtide. However, the biacore data presented by the authors do not support this statement. An estimate of the affinity based on the data provided suggests that the Tyr126 mutant is greatly affected in its affinity for PIFtide, probably well over 30 fold decreased affinity, perhaps giving up to 100 fold less binding. Thus, we cannot agree with Komander, D. et al., J. Biol. Chem. 280:18797-18802 (2005) conclusion from the Tyr126 work. It was suggested that the mutation of Tyr126 would have such effect, by losing its ability to interact with Ser241. However, we did not observe the interaction with Ser241 in the three distinct crystal forms we obtained in this project. Thus, on the basis of the structural data presented here, a possible role for Tyr126 may be to correctly position the ATP molecule for catalysis.

**Table 4: Data collection and refinement statistics of the structures PDK1_30 and PDK1_33.**

| Dataset | PDK1_30 | PDK1_33 |
|---|---|---|
| Complex: PDK1*dm* + atp + | ATP + PIF | AMP-PCP + PKB-HM |
| *A. Data collection* | | |
| Beamline (ESRF) | ID23-1 | ID14-3 |
| Wavelenght(Å) | 1,0732 | 1,0732 |
| Space group | C2 | P2(1) |
| Number of molecules in ASU | 2 | 4 |
| Unit cell dimensions | | |
| a (Å) | 98.51 | 47.46 |
| b (Å) | 171.40 | 169.69 |
| c (Å) | 47.73 | 95.47 |
| β (α, γ = 90°) | 92.84 | 92.90 |
| Resolution (Å) | 47.7-3.2 (3.4-3.2) | 47.7-2.2 (3.3-2.2) |
| Unique reflections | 13,025 | 76,275 |
| Completeness (%) | 99.9 (99.8) | 100 (100) |
| Multiplicity | 5.5 (5.5) | 3.6 (3.6) |
| Mean (I / σ (I)) | 15.2(6.8) | 12.8(3.9) |
| R_{merge} | 0.082 (0.223) | 0.070 (0.364) |
| | | |
| *B. Model refinement* | | |
| Reflections used | 12,365 | 72,420 |
| R-factor | 0.191 | 0.204 |
| R_{free} | 0.243 | 0.248 |
| Refined non-H protein atoms | 4501 | 9196 |
| Refined solvent atoms | 0 | 304 |
| r.m.s.d. from ideal | | |
| Bond lenghts (Å) | 0.015 | 0.016 |
| Bond angles (deg) | 1.658 | 1.709 |
| Mean B-values (Å²) | 50.553 | 17.268 |

## Claims

1. A mutant protein kinase derived from a starting protein kinase having a hydrophobic pocket in the position equivalent to the hydrophobic PIF-binding pocket defined by the residues Lys115, Ile118, Ile119, Val124, Val124, Val127 and/or Leu155 of full length human PDK1 shown in SEQ ID NO:1 and having a phosphate binding pocket equivalent to the phosphate binding pocket defined by the residues Lys76, Arg131, Thr148 and/or Gln150 of full length human PDK1 shown in SEQ ID NO:1, wherein said mutant protein kinase has a at least two mutations in one of its motives equivalent to AGNEYLIFQK (SEQ ID NO:2) and LDHPFFVK (SEQ ID NO:3) of human PDK1, or a fragment or derivate thereof.

2. The mutant protein kinase of claim 1, wherein the starting protein kinase is derived from a mammalian protein kinase grouped within the AGC group of protein kinases, such as SGK, PKB, S6K, MSK, RSK, LAT, NDR, MAST, ROCK, DMPK, MRCK, PKA, PKG, GRK, PRK, PKC and their isoforms, or Aurora or YANK protein kinases and their isoforms, preferably is derived from a PDK1 protein kinases from infectious organisms such as Candida spieces such as Candida albicans PDK1, , *Aspergillus* spp., *Cryptococcus neoformans, Histoplasma capsulatum,* or *Coccidioides,* most preferably is derived from the human protein kinase PDK1 having SEQ ID NO: 1.

3. The mutant protein kinase of claim 1 or 2, wherein the mutation in the motif of SEQ ID NO:2
(i) is a non-conservative mutation; and/or
(ii) is a mutation of the residues Y or Q, preferably said motif has the mutation of the residue Y with G or a mutation of the residue Q to A, most preferably said motif has the Y to G and Q to A mutations.

4. The mutant protein kinase of claim 1 or 2, wherein the mutation in the motif of SEQ ID NO:3
(i) is a non-conservative mutation; and/or
(ii) is a mutation of the residues D, H, P, or K preferably said motif has the mutation of the residue D or K with M, H or P.

5. The mutant protein kinase of any one of claims 1 to 4, wherein said starting protein kinase is human PDK1 shown in SEQ ID NO:1 and said mutant protein kinase has at least two mutations at a position corresponding to positions Tyr288 Gln292, and may have one or more further point mutations at positions corresponding to Lys296, Asn296 and Ile295, wherein the numbering refers to the full length human PDK1 shown in SEQ ID NO:1, or a fragment or derivate thereof.

6. The mutant protein kinase of any one of claims 1 to 5, wherein
(i) the fragment of the mutated protein kinase is C- and/or N-terminally truncated and comprises the hydrophobic PIF-binding pocket, the phosphate binding pocket and the motives of SEQ ID NOs: 2 and 3; and/or
(ii) the derivative of the mutated protein kinase is a C- and/or N-terminal fusion product with a peptide or protein sequence and/or with a low molecular chemical compound; and/or
(iii) the mutated protein kinase is in a crystalline form.

7. The mutant protein kinase of any one of claims 1 to 6, which has the mutations Tyr288 Gly and Gln292Ala, wherein the numbering refers to the full length human PDK1 shown in SEQ ID NO:1, preferably the mutant protein kinase has the the sequence shown in SEQ ID NOs: 4, 5 or 6.

8. A polynucleotide sequence encoding the mutated protein kinase of any one of claims 1 to 7.

9. A vector comprising the polynucleotide sequence of claim 8.

10. A host cell transformed with the vector of claim 9 and/or comprising the poynucleotide sequence of claim 8.

11. A process for producing the mutated protein kinase of claims 1 to 7 which comprises culturing the host cell of claim 10 and isolating said mutated protein kinase.

12. A method for identifying a compound that binds to the PIF-binding pocket allosteric site of a starting protein kinase as defined in claim 1 or 2, which comprises the step of determining the effect of the compound on the mutated protein kinase of claims 1 to 7 or the ability of the compound to bind to said mutated protein kinase.

13. The method of claim 12,
(i) wherein the protein kinase is an AGC kinase or other kinases possessing a similar regulatory site, preferably is a PDK1 kinase, most preferably is a human PDK1 kinase; and/or
(ii) which further comprises the step of determining the effect of the compound on the starting protein kinase of claim 1 or 2 or the ability of the compound to bind to said starting protein kinase; and/or
(iii) which further comprises adding a compound binding to the phosphate binding pocket.

14. A kit for performing the method of claim 12 or 13 which comprises a mutated proten kinase of any one of claims 1 to 7.

15. A compound identified by the method of claim 12 or 13 binding to the PIF-binding pocket allosteric site of a starting protein kinase.
